# EUROPÄISCHE PATENTSCHRIFT

(11) **EP 0 385 231 B1**
(45) Veröffentlichungstag und Bekanntmachung des Hinweises auf die Patenterteilung: **18.09.1996**
(21) Anmeldenummer: 90103204.5
(22) Anmeldetag: 20.02.1990
(51) Int. Cl.: A01N 37/32, A01N 43/38, A01N 43/80, A01N 43/84, C07D 209/48, C07D 413/04, C07D 413/14, C07D 403/04

(54) **Verwendung von Derivaten des N-Phenyl-3,4,5,6-tetrahydrophthalimids zur Desikkation und Abszission von Pflanzenorganen**
Use of derivatives of N-phenyl-3,4,5,6-tetrahydrophthalimide in the desiccation and abscission of plant organs
Utilisation de dérivés du N-phényl-3,4,5,6-tétrahydrophtalimide pour la dessication et l'excision d'organes de plantes

(30) Priorität: 25.02.1989 DE 3905916
(43) Veröffentlichungstag der Anmeldung: 05.09.1990
(73) Patentinhaber: BASF Aktiengesellschaft, 67063 Ludwigshafen (DE)
(72) Erfinder: Grossmann, Klaus, Dr., D-6703 Limburgerhof (DE); Mulder, Christiaan E.G., Dr., Nelspruit 1200 (ZA); Wuerzer, Bruno, Dr., D-6701 Otterstadt (DE)

(56) Entgegenhaltungen:
- EP-A- 0 207 894
- EP-A- 0 263 299
- EP-A- 0 296 416
- EP-A- 0 300 398
- EP-B- 0 170 191
- EP-B- 0 177 032
- WO-A-87/04049
- DE-A- 3 714 373
- DE-A- 3 807 295

## Beschreibung

Die vorliegende Erfindung betrifft die Verwendung von Derivaten des N-Phenyl-3,4,5,6-tetrahydrophthalimids der allgemeinen Formeln I und/oder II 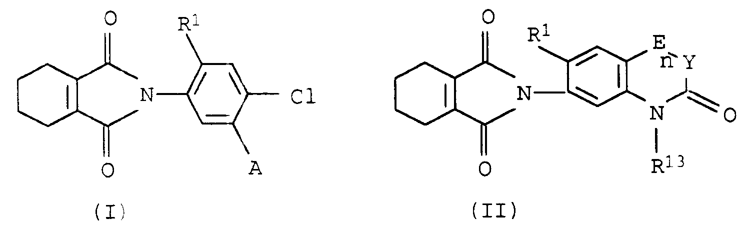 in denen die Substituenten folgende Bedeutung haben:
- R¹: Wasserstoff, Fluor oder Chlor;
- A: eine Gruppe I-1 bis I-7 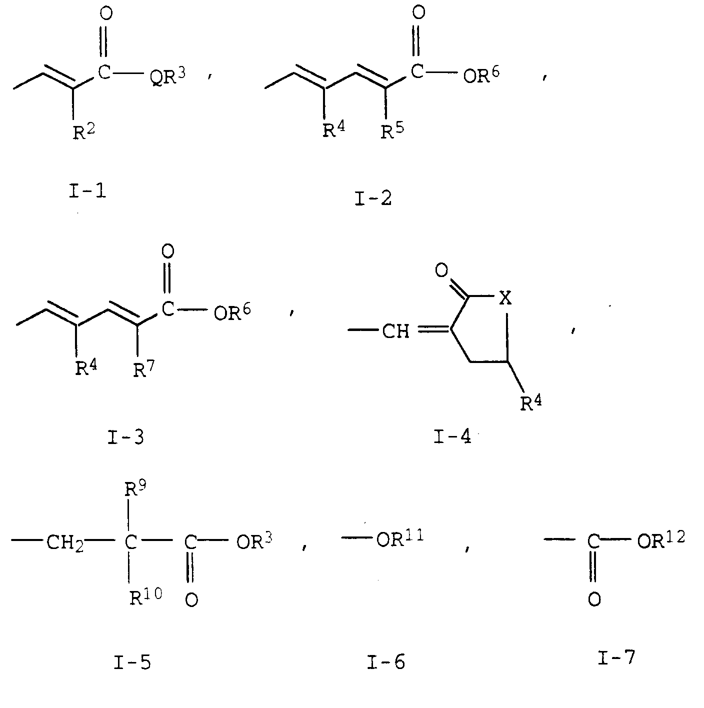
- R²: Wasserstoff, Chlor, Brom, Cyano oder C₁-C₆-Alkyl;
- R³: Wasserstoff, C₁-C₈-Alkyl, C₂-C₈-Alkenyl, C₂-C₄-Alkinyl, C₁-C₄-Alkoxy-C₁-C₄-alkyl, C₁-C₄-Alkylthio-C₁-C₄-alkyl, Phenyl-C₁-C₃-alkyl und im Fall von Q = NR⁸ darüber hinaus C₁-C₄-Hydroxyalkyl, C₁-C₄-Alkoxy, C₂-C₄-Alkenyloxy, Phenyl, Phenyl substituiert durch Halogen, C₁-C₄-Alkyl oder -Alkoxy, C₁-C₄-Halogenalkyl oder -Halogenalkoxy;
- R⁴, R⁵: Wasserstoff oder C₁-C₃-Alkyl;
- E: Sauerstoff oder Methylen;
- X: Sauerstoff oder Schwefel;
- Q: Sauerstoff, Schwefel oder NR⁸;
- Y: Sauerstoff, Schwefel, CHR⁴;
- n: die Zahl 0 oder 1;
- R⁶: Wasserstoff, C₁-C₆-Alkyl, C₁-C₆-Alkoxyalkyl, C₁-C₆-Alkylthioalkyl oder C₅-C₆-Cycloalkyl;
- R⁷: Wasserstoff oder C₁-C₄-Alkoxycarbonyl;
- R⁸: C₁-C₈-Alkyl, C₃-C₄-Alkenyl, C₃-C₄-Alkinyl oder C₁-C₆-Alkoxyalkyl;
- R⁹: Wasserstoff, Chlor, Brom, Cyano, C₁-C₄-Alkyl, C₃-C₄-Alkenyl, C₃-C₄-Alkinyl, C₁-C₆-Alkoxyalkyl, C₁-C₆-Alkylthioalkyl oder Cyclohexylmethyl;
- R¹⁰: Wasserstoff, Halogen, Cyano, C₁-C₄-Alkyl, C₁-C₃-Alkylcarbonyl, C₁-C₄-Alkoxycarbonyl oder R⁹ und R¹⁰ zusammen eine C₄-C₅-Alkylen- oder C₄-C₅-Oxoalkylengruppe;
- R¹¹: C₁-C₄-Alkyl, C₃-C₄-Alkenyl, C₃-C₄-Alkinyl, C₁-C₄-Alkylcarbonyl, C₁-C₄-Alkoxycarbonyl-C₁-C₄-alkyl, Tetrahydrofurfuryl, Dihydropyranylmethyl, Dihydrothiopyranylmethyl, Tetrahydropyranylmethyl oder Tetrahydrothiopyranylmethyl,
- R¹²: Wasserstoff, C₁-C₄-Alkyl, C₃-C₄-Alkinyl, C₁-C₄-Alkoxycarbonyl-C₁-C₄-alkyl oder -N=C(CH₃)₂;
- R¹³: Wasserstoff, C₁-C₄-Alkyl, C₃-C₄-Alkenyl, C₃-C₄-Alkinyl, Benzyl, mit Halogen oder C₁-C₄-Alkyl ein- bis dreifach substituiertes Benzyl,
zur Desikkation und Abszission von Pflanzenorganen von Kulturpflanzen.

Weiterhin betrifft die Erfindung ein Verfahren zur Desikkation und Abszission von Pflanzenorganen, insbesondere der Blätter, mittels der o.g. Verbindungen I und II.

Aus EP-A 207 894 und DE-A 37 14 373 geht hervor, daß speziell substituierte N-Phenyl-3,4,5,6-tetrahydrophthalimide neben einer selektiven Herbizideigenschaft auch eine das Pflanzenwachstum hemmende Wirkung aufweisen. Für die Verbindungen der DE-A 37 14 373 werden ferner wachstumsregulatorische Eigenschaften angegeben, darunter beispielhaft das Auslösen von Desikkation, Abszission und Wuchsstauchung. In der EP-A 207 894 wird anhand eines Ausführungsbeispiels auf die Desikkations- und Defoliationswirkung bei Baumwollpflanzen hingewiesen, ohne jedoch die mit Aufwandmengen von 1,2, 0,6 oder 0,3 kg/ha getesteten Wirkstoffe zu spezifizieren. Über die Wirkung der Testsubstanzen bei 0,3 kg/ha wurden keine Angaben gemacht.

In der EP-A-0 296 416 werden u.a. Verbindungen der Formel Ia 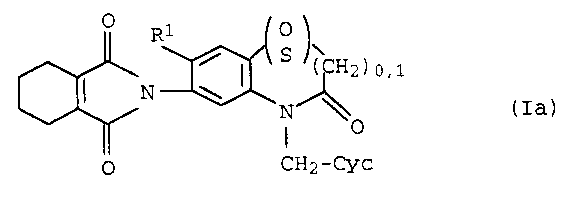 beschrieben, wobei Cyc für C₃-C₆-Cycloalkyl, gegebenenfalls substituiertes 5-gliedriges Heterocyclyl oder 6-gliedriges Heteroaryl mit 1 bis 3 N-Atomen steht. Gemäß der Lehre dieser Schrift sind die Verbindungen neben ihrer besonderen Eignung als Vorauflauf-Herbizide auch als Entlaubungsmittel brauchbar.

Die Anwendung von Tetrahydrophthalimidderivaten als Herbizide ist in einer Reihe von Publikationen beschrieben, beispielsweise in DE-A-36 03 789, DE-A-36 07 300, DE-A-30 13 162, DE-A-31 09 035, DE-A-35 33 440, EP-A-61 741, EP-A-83 055, EP-A-68 822, EP-A-236 916, GB-A-20 71 100, U.S. 3,878,224, JP-A 59/155 358 und JP-A 61/027 962. Aus diesen Veröffentlichungen geht die Verwendung der Verbindungen als Abszissionsmittel zur gezielten Induktion des Abwurfes von Blättern, Blüten oder Früchten bei Kulturturpflanzen, z.B. Baumwolle, Citrus, Oliven und Kern- und Steinobst, sowie ihre Verwendung als Desikkantien zur Austrocknung der oberirdischen Teile in Kulturpflanzen, z.B. Kartoffel, Raps, Sonnenblume und Sojabohne, nicht hervor.

Sowohl an Abszissions- als auch Desikkationsmitteln besteht ein starkes wirtschaftliches Interesse aus Gründen der Ernteerleichterung. Insbesondere im intensiven Baumwollanbau ist die Anwendung von Entblätterungsmitteln (Defoliantien) Grundvoraussetzung für einen effektiven Einsatz von Pflückmaschinen für die Kapselernte. Die bisher verwendeten Wirkstoffe können nicht immer voll befriedigen.

Es wurde nun gefunden, daß die eingangs definierten N-Phenyl-3,4,5,6-tetrahydrophthalimide eine ausgeprägte Wirksamkeit hinsichtlich der Abszission und Desikkation von Pflanzenorganen besitzen. Ihre Verwendung bietet gegenüber bekannten Mitteln erhebliche Vorteile:
a) ist ihre Wirkung bereits bei geringen Aufwandmengen von ca. 60 bis 250 g/ha optimal,
b) wirken sie bei vergleichbarer Aufwandmenge wesentlich vollständiger,
c) ist ihre Wirkung auch bei niedrigen Temperaturen wesentlich sicherer.

Neben ihrer hervorragenden Wirkung als Defoliantien zeigen die Verbindungen I und/oder II eine sehr gute Wirksamkeit bei ihrer Anwendung als Desikkantien zur Austrocknung der oberirdischen Pflanzenteile bei Kulturpflanzen wie Kartoffel, Sonnenblume, Sojabohne und Raps, um den Erntevorgang zu erleichtern. Außerdem führen sie zu einer gleichmäßigen Abreife der Erntefrüchte.

Im Hinblick auf ihre Wirksamkeit besonders bevorzugte Verbindungen sind N-Phenyl-3,4,5,6-tetrahydrophthalimide der Struktur I, in denen R¹ Fluor und insbesondere Wasserstoff darstellt.

Bevorzugte Reste A sind die folgenden Gruppen: 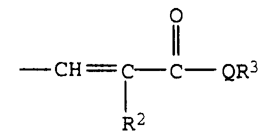 mit Q = Sauerstoff, R² = H, Cl, Br, CN und C₁-C₆-, insbesondere C₁-C₄-Alkyl und R³ = H, C₁-C₈-, insbesondere C₁-C₄-Alkyl wie Methyl, Ethyl, Propyl, i-Propyl, n-Butyl, i-Butyl, C₂-C₈-, insbesondere C₂-C₄-Alkenyl, C₃- oder C₄-Alkinyl, C₁-C₈-Alkoxyalkyl, insbesondere C₁-C₄-Alkoxy-C₁-C₄-alkyl, C₁-C₈-Alkylthioalkyl, insbesondere C₁-C₄-Alkylthio-C₁-C₄-alkyl, C₁-C₁₇-Aralkyl, z.B. Phenylalkyl wie Benzyl oder 2-Phenylethyl, C₃-C₆-, insbesondere C₅- oder C₆-Cycloalkyl wie Cyclopentyl oder Cyclohexyl;

OR¹¹, 1-6

worin R¹¹ insbesondere Tetrahydrofurfuryl, Dihydropyranylmethyl, Dihydrothiopyranylmethyl, Tetrahydropyranylmethyl oder Tetrahydrothiopyranylmethyl bedeutet.

Die N-substituierten Tetrahydrophthalimide I sind aus 3,4,5,6-Tetrahydrophthalsäureanhydrid und entsprechend substituierten Anilinderivaten, die durch Reduktion der entsprechenden Nitroverbindungen erhältlich sind, zugänglich. In der Regel wird die Umsetzung in einem inerten Lösungsmittel bei Temperaturen von 20 bis 200°C, vorzugsweise 40 bis 150°C durchgeführt. Als Lösungsmittel eignen sich z.B. niedere Alkancarbonsäuren wie Eisessig und Propionsäure oder aprotische Lösungsmittel wie Toluol und Xylol in Gegenwart von sauren Katalysatoren, z.B. aromatischen Sulfonsäuren.

N-Phenyl-tetrahydrophthalimide, in denen A die Gruppe I-1 darstellt sind aus der DE-A 36 03 789 (EP-A 240 659) oder DE-A 37 24 399 (EP-A-300 387) bekannt. Sie können auch dadurch hergestellt werden, daß man einen Aldehyd der Formel IV 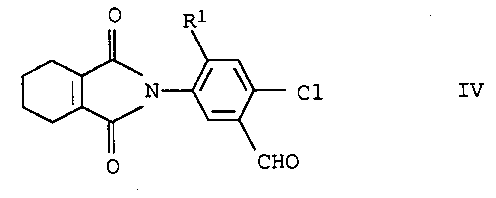 mit einem Phosphoran der Formel V 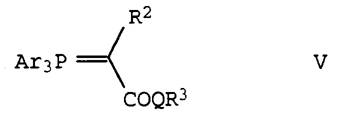 worin Ar einen unsubstituierten oder substituierten Phenylrest bedeutet, bei Temperaturen von -10 bis 100°C und in Gegenwart eines Lösungsmittels umsetzt. Die als Ausgangsmaterial verwendeten Aldehyde der allgemeinen Formel IV sind nach den Methoden der deutschen Patentanmeldung DE-A 38 15 042 auf einfache Weise zugänglich. Der Rest R dieser Verbindungen kann für Wasserstoff oder Fluor stehen.

Die zur Herstellung der Tetrahydrophthalimide benötigten Phosphorane V, welche auch als Phosphor-Ylide bezeichnet werden, sind nach literaturbekannten Methoden (z.B. Houben-Weyl, Methoden der Organischen Chemie, Ed. El, S. 636-639, Georg-Thieme Verlag, Stuttgart 1982) erhältlich.

Die Umsetzung der Ausgangsverbindungen IV und V wird im allgemeinen vorteilhaft in Gegenwart eines Lösungsmittels durchgeführt. Als Lösungsmittel können alle üblicherweise bei der Durchführung von Wittig-Reaktionen verwendeten Lösungsmittel Anwendung finden, beispielsweise halogenierte Lösungsmittel wie Chloroform, oder Ether wie Tetrahydrofuran, Dioxan und Ethylenyglykoldimethylether. Bevorzugte Lösungsmittel sind Alkohole, insbesondere C₁- bis C₄-Alkohole wie Methanol, Ethanol, Propanol, Isopropanol, Butanol, Isobutanol und tert.-Butanol. Die Lösungsmittel können auch in Form von Lösungsmittelgemischen eingesetzt werden, in der Regel werden jedoch bevorzugt die reinen Lösungsmittel verwendet. Dienen Alkohole als Lösungsmittel, so findet im allgemeinen vorteilhaft derjenige Alkohol als Lösungsmittel Verwendung, der der Alkoholkomponente der Estergruppe in I und V entspricht.

Es versteht sich von selbst, daß die optimale Reaktionstemperatur von den jeweils umzusetzenden Ausgangsverbindungen IV und V sowie dem verwendeten Lösungsmittel abhängig ist. Im allgemeinen wird die Reaktion jedoch bei Temperaturen zwischen -10°C und 100°C, bevorzugt zwischen -10°C und 60°C und besonders vorteilhaft zwischen 10°C und 40°C ausgeführt.

Die Ausgangsverbindungen IV und V können in stöchiometrischen Mengen miteinander umgesetzt werden. Es kann sich jedoch als vorteilhaft erweisen, wenn einer der beiden Reaktanten, IV oder V, in einem molaren Überschuß von 10 bis 20 % in die Reaktion eingesetzt wird.

N-Phenyl-tetrahydrophthalimide mit der Gruppe A = I-2 oder I-3 sind in der DE-A-36 03 789 (EP-A-240 659) offenbart. N-Phenyltetrahydrophthalimide mit A = I-4 sind in der deutschen Anmeldung DE-A 38 19 464 beschrieben. Verbindungen I mit A = I-5 sind in der DE-A-37 24 395 (EP-A-300 398) offengelegt. Verbindungen I mit A = I-6 sind in der deutschen Anmeldung DE-A-37 36 297 beschrieben. Verbindungen I mit A = I-7 sind aus den DE-A-31 09 035 und 35 33 440 und aus GB-A-20 71 100 bekannt.

N-Aryltetrahydrophthalimidverbindungen der Struktur II können erhalten werden, indem man ein entsprechend substituiertes Amin III 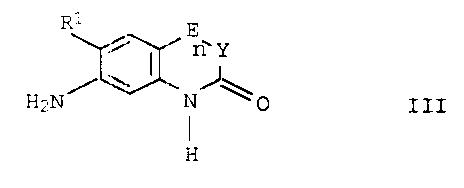 mit einer N-alkylierenden Verbindung Z-R¹³ umsetzt, wobei Z einen die N-Alkylierung auslösenden Säurerest bedeutet und das erhaltene Amin in an sich bekannter Weise mit Tetrahydrophthalsäureanhydrid reagieren läßt. Man kann auch zuerst die Umsetzung von III mit Tetrahydrophthalsäureanhydrid durchführen und anschließend N-alkylieren. Als Alkylierungsmittel kommen bevorzugt Halogenverbindungen, Tosylate oder Mesylate der einzuführenden Reste in Betracht. Die Herstellung von Verbindungen der Struktur II ist z.B. in der deutschen Anmeldung DE-A-38 07 295 beschrieben.

Die Wirkstoffe I und/oder II können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel wie Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Napthalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Umhüllungs-, Imprägnierungs- und Homogengranulate, können durch Bindung der Wirkstoffe an feste Tragerstoffe hergestellt werden. Feste Trägerstoffe sind Mineralerden wie Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver und andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:
I. Man vermischt 90 Gew.-Teile der Verbindung gemäß Beispiel l.l mit 10 Gew.-Teilen N-Methyl-α-pyrrolidon und erhält eine Lösung, die zur Anwendung in Form kleinster Tropfen geeignet ist.
II. 20 Gew.-Teile der Verbindung Nr. 1.17 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
III. 20 Gew.-Teile der Verbindung Nr. 6.1 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
IV. 20 Gew.-Teile der Verbindung Nr. 1.26 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.
V. 3 Gew.-Teile der Verbindung Nr. 1.26 werden mit 97 Gew.-Teilen feinteiligem Kaolin vermischt. Man erhält auf diese Weise ein Stäubemittel, das 3 Gew.% des Wirkstoffs enthält.
VI. 30 Gew.-Teile der Verbindung Nr. 1.17 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VII. 20 Gew.-Teile der Verbindung Nr. 1.17 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensates und 68 Teilen paraffinischen Mineralöls innig vermischt. Man erhält eine stabile ölige Dispersion.

Eine Förderung des Wirkung und der Wirkungsgeschwindigkeit kann z.B. durch wirkungssteigernde Zusätze wie organische Lösungsmittel, Netzmittel und Öle erzielt werden. Das läßt eine Minderung der Aufwandmenge des eigentlichen Wirkstoffes zu.

Je nach gewünschtem Effekt, Pflanzenspecies, Entwicklungsstadium der zu behandelnden Pflanzen und einzusetzendem Mittel wird die Aufwandmenge der einzelnen Wirkstoffe variiert.

Die Mittel werden den Pflanzen vornehmlich durch Blattspritzung zugeführt. Dabei kann die Ausbringung z.B. mit Wasser als Trägerstoff durch übliche Spritztechniken mit Spritzbrühenmengen von etwa 100 bis 1000 l/ha erfolgen. Eine Anwendung der Mittel im sogenannten "Low Volume"- und "Ultra-low-Volume"-Verfahren ist ebenso möglich wie ihre Applikation in Form von sogenannten Mikrogranulaten.

Die erfindungsgemäßen Mittel können in Aufwandmengen von 0,001 bis 5 kg/ha, vorzugsweise 0,01 bis 3 kg/ha, insbesondere 0,01 bis 0,6 kg/ha, eingesetzt werden.

Die Mittel können entweder für sich allein, in Mischung mit anderen Mitteln oder mit anderen Wirkstoffen angewendet werden. Gegebenenfalls können andere Entblätterungs-, Desikkations-, Pflanzenschutz- oder Schädlingsbekämpfungsmittel je nach dem gewünschten Zweck zugesetzt werden.

Auch hat sich gezeigt, daß Mischungen der erfindungsgemäßen Mittel z.B. mit den nachfolgend aufgeführten Wirkstoffen (A.)-(C.) zu einer weiteren Förderung der Desikkations- und Entblätterungswirkung führen und zu einer besseren Bekämpfung des unerwünschten Wiederaustriebs von Pflanzen nach der Desikkation bzw. Entblätterung (insbesondere bei Baumwolle) beitragen:
(A.) Herbizidaktive Wirkstoffe aus der Gruppe der
   a. Chloracetanilide, z.B. das in DE-A-26 48 008 beschriebene 2-Chlor-N-(2,6-dimethylphenyl)-N-(1H-pyrazol-1-yl-methyl)-acetamid (common name: Metazachlor),
   b. Substituierte Chinolin-8-carbonsäuren, z.B. die in EP-A-60 429 beschriebene 3,7-Dichlor-chinolin-8-carbonsäure (common name: Quinchlorac) und die in EP-A-104 389 beschriebene 3-Methyl-7-chlor-chinolin-8-carbonsäure (common name: Quinmerac),
   c. Cyclohexenonderivate, z.B. das in DE-A-28 22 304 beschriebene 2[(1-Ethoxyimino)butyl]-5-[2-(ethylthio)-propyl]-3-hydroxy-2-cyclohexen-1-on (common name: Sethoxydim) und das in DE-A-31 21 355 beschriebene 2-[1-(Ethoxyimino)butyl]-3-hydroxy-5-(2H-tetrahydrothiopyran-3-yl)-2-cyclohexen-1-on (common name: Cycloxydim),
   d. Phenoxyalkancarbonsäuren, z.B. (4-Chlor-2-methyl-phenoxy)essigsäure,
   e. 3-(Isopropyl)-1H-2,1,3-benzothiadiazin-4(3H)-on 2,2-dioxid, beschrieben in DE-A-15 42 836 (Bentazon®),
   f. Dinitroaniline, z.B. das in DE-A-22 41 408 beschriebene N-(1-Ethylpropyl)-3,4-dimethyl-2,6-dinitro-anilin,
   g. Imidazolinone, z.B. 2-[4,5-Dihydro-4-methyl-4-(1-methylethyl)-5-oxo-1H-imidazol-2-yl]-3-chinolincarbonsäure (Scepter®, common name: Imazaquin); 2-(4-Isopropyl-4-methyl-5-oxo-2-imidazolin-2-yl)-nikotinsäure kombiniert mit Isopropylamin im Verhältnis 1:1 (Arsenal®, common name: Imazapyr); 2-[4,5-Dihydro-4-methyl-4-(1-methylethyl)-5-oxo-1H-imidazol-2-yl)-5-ethyl-3-pyridincarbonsäure (Pursuit®, common name: Imazethapyr); Methyl-2-(4-isopropyl-4-methyl-5-oxo-2-imidazolin-2-yl)-p-toluat kombiniert mit Methyl-6-(4-isopropyl-4-methyl-5-oxo-2-imidazolin-2-yl)-m-toluat (Assert®, common name: Imazamethabenz) sowie Imazamethapyr (common name; trade mark: Cadre®),
   h. Sulfonylharnstoffderivate, z.B. die in den folgenden Tabellen 1 und 2 aufgeführten Verbindungen und der als DPX-E 9636 bekannte 1-(4,6-Dimethoxypyrimidin-2-yl)-3-(ethylsulfonyl)-2-pyridylsulfonyl)harnstoff. 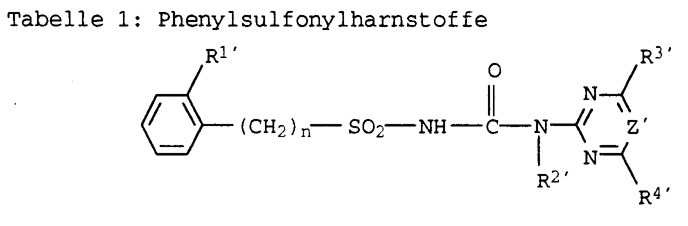 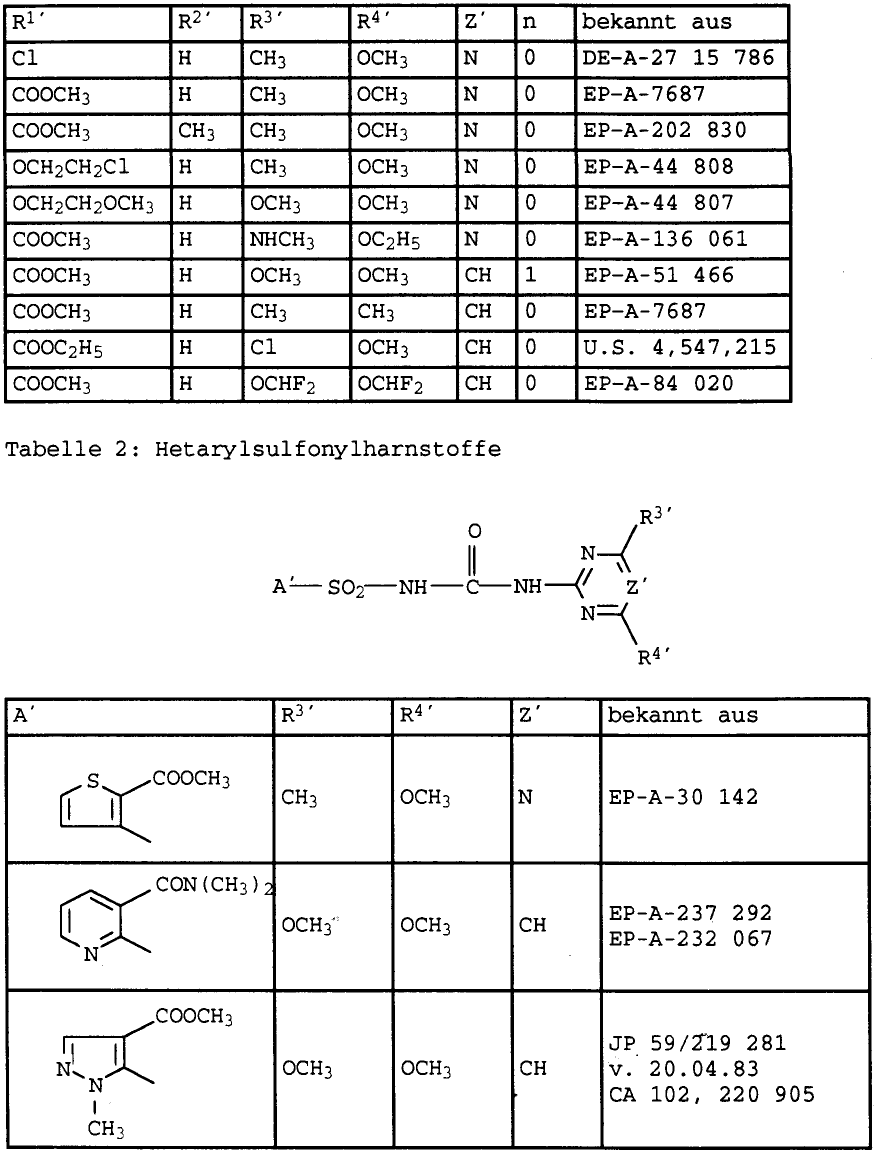
   Die in den Tabellen 1 und 2 aufgeführten Handelsprodukte sind z.B. unter den Handelsnamen Glean®, Allyl®, Express®, Logran®, Setoff®, Muster®, Londax®, Oust®, Classic®, Beacon®, Harmony® oder Remedy® bekannt.
   i. Diphenyletherderivate wie die in DE-A-23 11 638 und EP-A-40 898 beschriebenen 5-[2-Chlor-4-(trifluormethyl)phenoxy]-2-nitrobenzoesäure und dessen Salze (Acifluorfen) sowie 5-[2-Chlor-4-(trifluormethyl)-phenoxy]-2-nitrobenzoesäure-(ethoxycarbonylmethyl)ester (Fluoroglycofen) sowie die Diphenylether 1-(Carboethoxy)ethyl-5-[2-chlor-4-(trifluormethyl)-phenoxy]-2-nitrobenzoat (common name: Lactofen, Cobra®); 2-Chlor-1-(3-ethoxy-4-nitrophenoxy)-4-trifluormethylbenzol (common name: Oxyfluorfen) und 5-(2-Chlor-4-trifluormethyl)phenoxy)-N-methylsulfonyl-2-nitrobenzamid (common name: Fomesafen, Flex®, EP-A-3 416).
(B.) Defoliantien und Desikkantien, wie z.B. genannt in G.W. Cathey, (1986) Physiology of defoliation in cotton production, in "Cotton Physiology" (J.R. Mauney, J. McD. Stewart, eds.) The Cotton Foundation reference book series, No. 1, Chapter 14, 143-153, in Morgan, P.W. (1985) Chemical manipulation of abscission and desiccation. In "Agricultural Chemicals of the Future" (J.L. Hilton, ed.) BARG Symposium 8, 61-74. Rowman & Allanheld, Totowa, in R. Krämer (1989) Erstellung leistungsfähiger Kernobstjungpflanzen, Dissertation der Hohen Landwirtschaftlichen Fakultät der Universität Bonn und in H. Bergmann, D. Martin (1989) Chemical manipulation of desiccation and defoliation and essential aspects for the application and development of new chemical compounds in the future, in "Chemistry of Plant Protection 2" (G. Haug, H. Hoffmann, eds.) 197-246, Springer-Verlag Berlin Heidelberg.
   a. Harnstoff-Derivate, wie z.B. der aus DE-OS 25 06 690 und 26 19 861 bekannte N-Phenyl-N'-1,2,3-thiadiazol-5-yl-harnstoff, der in DE-OS 36 12 830 beschriebene N-Phenyl-N'-1,3,4-thiadiazol-2-yl-harnstoff oder der in DE-OS 28 43 722 beschriebene N-Phenyl-N'-2-chlorpyrid-3-yl-harnstoff oder der oben erwähnte 3-(3-Chlor-4-methoxyphenyl)-1,1-dimethylharnstoff (common name: Metoxuron),
   b. (2-Chlorethyl)phosphonsäure (Ethrel®),
   c. S,S,S-Tributylphosphortrithioat und S,S,S-Tributylphosphortrithioit,
   d. 2,3-Dihydro-5,6-dimethyl-1,4-dithiin-1,1,4,4-tetraoxid (Harvade®),
   e. Salze des N-(Phosphonomethyl)glycins, wie das Isopropylammoniumsalz (Roundup®),
   f. Ammonium-DL-homoalanin-4-yl-(methyl)-phosphinat (Glufosinat-Ammonium),
   g. Magnesium- und Natriumchlorat,
   h. Ammoniumsulfat, -nitrat,
   i. Hydrogencyanamid, Calciumcyanamid,
   j. Kaliumiodid,
   k. Cu-, Fe-Ethylendiamintetraacetat,
   l. Arsensäure und deren Derivate, wie das Hydroxydimethylarsinoxid (common name: Dimethylarsinic acid),
   m. 1,2-Dihydropyridazin-3,6-dion,
   n. 7-Oxabicyclo[2,2,1]heptan-2,3-dicarbonsäure (common name: Endothall),
   o. 6,7-Dihydrodipyridol (1,2-α:2',1'-c)pyridilium-Ion als Dibromid-Monohydrat Salz (common name: Diquat) und 1,1'-Dimethyl-4,4'-bipyridinium-Ion als Dichlorid- oder Dimethylsulfat-Salz (common name: Paraquat),
   p. 3,5-Dibrom- bzw. Diiod-4-hydroxybenzonitril (common name: Bromoxynil),
   q. substituierte Dinitrophenole, wie das 2-sec-Butyl-4,6-dinitrophenol (common name: Dinoseb),
   r. Triazinderivate, wie das 2-Ethylamino-4-isopropylamino-6-methylthio-1,3,5-triazin (common name: Ametryne),
   s. Triazolderivate, wie das 1H-1,2,4-Triazol-3-ylamin (common name: Amitrol),
   t. Benzothiazole, wie das 2-n-Butylmercapto-benzothiazol (common name: Butylcaptax),
   u. Di-n-butyl-1-n-butylaminocyclohexylphosphonat (common name: Buminafos)
(C.) Wachstumsretardantien aus der Gruppe
   a. Quartäre Ammoniumsalze aus der Gruppe der
      N,N-Dimethyl-azacycloheptaniumsalze,
      N,N-Dimethylpiperidiniumsalze,
      N,N-Dimethylhexahydropyridaziniumsalze,
      N,N-Dimethyl-tetrahydropyridaziniumsalze,
      N-Methyl-pyridiniumsalze, N,N-Dimethyl-pyrrolidiniumsalze und N,N,N-Trimethyl-N-2-chlorethylammoniumsalze,
      insbesondere das N-2-Chlorethyl-N-trimethylammoniumchlorid (Common name: Chlormequatchlorid) und das
      N,N-Dimethylpiperdiniumchlorid (Common name: Mepiquatchlorid),
   b. Pyrimidinverbindungen wie aus US 3 818 009 und aus Journal of Plant Growth Regulation 7:27, 1988, bekannt (z.B. die mit dem common name: Ancymidol oder Flurprimidol),
   c. Pyridinverbindungen die aus DE-A-30 15 025 bekannt sind,
   d. Norbornadiazetine, wie sie in den DE-OS 26 15 878 und 27 42 034 beschrieben sind,
   e. Wachstumsregulatorisch wirksame Triazolverbindungen wie sie in der europäischen Anmeldung 88104320.2, in British Crop Protection Conference - Weeds 1982, Vol. 1, BCPC Publications, Croydon, 1982, Seite 3, in Plant Cell Physiol. 25, 611, in Pestic. Sci. 19, 153, in J. Agron. Crop. Sci. 158, 324 oder in J. Plant Growth Regul. 4, 181, beschrieben sind, z.B. 1-Phenoxy-3-(1H-1,2,4-triazol-1-yl)-4-hydroxy-5,5-dimethylhexan,
   f. 2-Acyl-3-hydroxycyclohex-2-en-1-one wie z.B. in EP-A-126 713 oder 123 001 beschrieben,
   g. 1-(4-Chlorphenoxy)-3,3-dimethyl-1-[1,2,4-triazol-1-yl]-butan -2-on (common name: Triadimefon),
      N-[2,4-Dimethyl-5-[trifluormethyl-sulfonylamino]phenylacetamid (common name: Mefluidide)
      2-Chlor-2',6'-diethyl-N-[methoxymethyl]-acetanilid (common name: Alachlor)
      S-Ethyl-dipropylthiocarbamat (common name: EPTC)
      Bernsteinsäure-2,2-dimethylhydrazid (common name: Daminozid)

Beispielsweise können folgenden Mischungspartner zugesetzt werden:
2-Methyl-6-ethyl-ethoxymethyl-2-chloracetanilid
2-Methyl-6-ethyl-N-(methoxy-1-methylethyl)-2-chloracetanilid
2-6-Dimethyl-N-(1-H-pyrazolyl-1-yl-methyl)-2-chloracetanilid
2,6-Diethyl-N-(methoxymethyl)-2-chloracetanilid
3-Methyl-7-chlorchinolin-8-carbonsäure (Salze, Ester)
3,7-Dichlor-chinolin-8-carbonsäure (Salze, Ester)
2-[(1-Ethoxyimino)butyl]-5-[2-(ethylthio)-propyl]-3-hydroxy-2-cyclohexan-1-on (Salze)
2-[(1-trans-chlorallyloxyimino)butyl]-5-[2-(ethylthio)-propyl]-3-hydroxy-2-cyclohexan-1-on (Salze)
2-[(1-trans-chlorallyloximino)propyl]-5-[2-(ethylthio)-propyl]-3-hydroxy-2-cyclohexan-1-on (Salze
2-[(1-Ethoximino)butyl]-5-[-2-H-tetrahydrothiopyran-3-yl)-3-hydroxy-2-cyclohexan-1-on (Salze)
2-[(1-Ethoximino)propyl]-5-(2,4,6-trimethylphenyl)-3-hydroxy-2-cyclohexan-1-on (Salze)
2-Methyl-4-chlorphenoxyessigsäure (Salze, Ester, Amide)
2-[2-Methyl-4-chlor-phenoxy]propionsäure (Salze, Ester, Amide)
4-[2-Methyl-4-chlor-phenoxy]-buttersäure (Salze, Ester, Amide)
4-[-2,4-Dichlorphenoxy]-buttersäure (Salze, Ester, Amide)
2-[-2,4-Dichlorphenoxy]-propionsäure (Salze, Ester, Amide)
2,4-Dichlorphenoxyessigsäure (Salze, Ester, Amide)
3,5,6-Trichlorpyridyl-2-oxyessigsäure (Salze, Ester, Amide)
3-(1-Methylethyl)-1-H-2,1,3-benzothiadiazin-4-(3H)-on-2,2-dioxid (Salze)
3-(1-Methylethyl)-1-cyan-2,1,3-benzothiadiazin-4-(3H)-on-2,2-dioxid (Salze)
N-(1-Ethylpropyl)-3,4-dimethyl-2,6-dinitro-anilin
2-[4,5-Dihydro-4-methyl-4-(1-methylethyl)-5-oxo-1H-imidazol-2-yl]-3-chinolincarbonsäure
2-(4-isopropyl-4-methyl-5-oxo-2-imidazolin-2-yl)nicotinsäure (Salze, Ester)
Methyl-3-[[[[(4-Methoxy-6-methyl-1,3,5-triazin-2-yl)amino]carbonyl]amino]sulfonyl]-2-thiophencarboxylat (Harmony®)
1-(4,6-Dimethoxypyrimidin-2-yl)-3-(ethylsulfonyl)-2-pyridylsulfonyl)harnstoff
5-[2-Chlor-4-(trifluormethyl)phenoxy]-2-nitrobenzoesäure (Salze)
N-Phenyl-N'-1,2,3-thiadiazol-5-yl-harnstoff
N-Phenyl-N'-1,3,4-thiadiazol-2-yl-harnstoff
N-Phenyl-N'-2-chlorpyrid-3-yl-harnstoff
N-3,4-(Dichlorphenyl)-N',N'-dimethylharnstoff
3-(3-chlor-4-methoxyphenyl)-1,1-dimethylharnstoff
2-chlorethylphosphonsäure
S,S,S-Tributylphosphortrithioat
S,S,S-Tributylphosphortrithioit
2,3-Dihydro-5,6-dimethyl-1,4-dithin-1,1,4,4-textraoxid
N-(Phosphonomethyl)glycine (Salze)
Ammonium-DL-homoalanin-4-yl-(methyl)-phosphinat
Magnesium- und Natriumchlorat
Ammoniumsulfat, -nitrat
Cyanamide
Kaliumjodit
Kupfer-, Eisenchelate
Arsensäure
Hydroxydimethylarsinoxid
1,2-Dihydropyridazin-3,6-dion
7-Oxabicyclo[2,2,1]-heptan-2,3-dicarbonsäure (Salze, Ester, Amide)
6,7-Dihydrodipyridol(1,2-α:2',1'-c)pyridilium-Ion als Dibromid-Monohydrat Salz
1,1'-Dimethyl-4,4'-dipyridinium-Ion als Dichlorid- oder Dimethylsulfat-Salz
3,5-Dibrom-4-hydroxybenzonitril
3,5-Dijod-4-hydroxybenzonitril
2-sec-Butyl-4,6-dinitrophenol
2-tert-Butyl-4,6-dinitrophenol
2-sec-Amyl-4,6-dinitrophenol
2-Ethylamino-4-isopropylamino-5-methylthio-1,3,5-triazin
1H-1,2,4-Triazol-3-ylamin
2-n-Butylmercapto-benzothiazol
Di-n-Butyl-1-n-butylaminocyclohexylphosphonat
N,N,N-Trimethyl-N-2-chlorethylammoniumsalze
N,N-Dimethylpiperidiniumsalze
N-Methylpyridiniumsalze
α-Cyclopropyl-α-(4-methoxyphenyl)-5-pyrimidin-methanol
α-Cyclopropyl-α-(4-trifluormethoxyphenyl)-5-pyrimidin-methanol
5(4-Chlorphenyl)3,4,5,9,10-pentaaza-tetracyclo[5,6,1,0²,⁶,0⁸,¹¹]-dodeca-3,9-dione
all-cis-8-(4-chlorphenyl)-3,4,8-triazatetracyclo[4,3,1,0,0²,⁵,0⁷,⁹]-dec-3-on
Bernsteinsäure-mono-N,N-dimethylhydrazid
N,N-Dipropylthiolcarbaminsäure-ethylester
N-2,4-Dimethyl-5-(trifluormethyl)-sulfonylamino-phenyl-acetamid
1-(4-Chlorphenoxy-)3,3-dimethyl-1-(1,2,4-triazol-1-yl)-2-butanon
2-Propylcarbonyl-5-ethoxycarbonyl-3-hydroxy-2-cyclohexen-1-on
1-(1,2,4-Triazol-1-yl)-1-methoxy-2-(2,4-dichlorphenyl)-propanol-2-
2,2-Dimethyl-4-(1,2,4-triazol-1-yl)-6-phenoxy-hexanol-3
2,2-Dimethyl-4-(1,2,4-triazol-1-yl)-5-(4-chlorphenyl)-pentanol-3-
2,2-Dimethyl-4-(1,2,4-triazol-1-yl)-5-(4-chlorphenyl)-penten-4-ol-1
2,2-Dimethyl-4-(1,2,4-triazol-1-yl)-5-cyclohexyl-penten-4-ol-3
1(5-Methyl-1,3-dioxan-yl-5)-4-(1,2,4-triazol-1-yl)-4-(4-trifluormethylphenyl)-propen-2-ol

Besonders bevorzugte N-Phenyl-3,4,5,6-tetrahydrophthalimide sind solche der Formel I, in der A für die Gruppe I-1 steht, in der Q Sauerstoff, R² Chlor oder Brom und R³ eine C₁-C₆-Alkylgruppe bedeutet. Hervorzuheben sind hier die Verbindungen Nr. 1.1 und insbesondere Nr. 1.17.

Bevorzugte Aufwandmengen für diese Verbindungen sind 0,02 bis 1,0, insbesondere 0,05 bis 0,5 kg/ha.

Bevorzugte Mischungspartner sind in der nachfolgenden Tabelle 3 aufgeführt, worin auch die jeweiligen Aufwandmengen der Mischungspartner angegeben sind. Die Gesamtaufwandmenge für die Mischung läßt sich aus der Summe der für die Tetrahydrophthalimide I angegebenen Menge und der in der Tabelle jeweils angegebenen Menge ermitteln.

**Tabelle 3**

| Mischungspartner | Aufwandmenge [kg/ha] |
|---|---|
| 3,7-Dichlor-chinolin-8-carbonsäure (Salze, Ester) (Quinclorac) | 0,05-0,5 |
| 2,4-Dichlorphenoxyessigsäure (Salze, Ester, Amide) | 0,05-1 |
| 2-[4,5-Dihydro-4-methyl-4-(1-methylethyl)-5-oxo-1H-imidazol-2-yl]-3-chinolincarbonsäure (Imazaquin) | 0,005-0,25 |
| Methyl-3-[[[[(4-Methoxy-6-methyl-1,3,5-triazin-2-yl)amino]carbonyl]amino]sulfonyl] -2-thiophen-carboxylat (Harmony®) | 0,001-0,1 |
| 1-(4,6-Dimethoxypyrimidin-2-yl)-3-(ethylsulfonyl)-2-pyridylsulfonyl)-harnstoff (DPX-E 9636) | 0,001-0,1 |
| 5-[2-Chlor-4-(trifluormethyl)phenoxy]-2-nitrobenzoesäure (Salze) (Acifluorfen) | 0,1-1 |
| N-Phenyl-N'-1,2,3-thiadiazol-5-yl-harnstoff (Thidiazuron) | 0,01-0,5 |
| (2-Chlorethyl)phosphonsäure (Ethephon) | 0,2-4 |
| S,S,S-Tributylphosphortrithioat (Def) | 0,2-2 |
| S,S,S-Tributylphosphortrithioit (Folex) | 0,2-2 |
| Natriumchlorat | 0,4-4 |
| 7-Oxabicyclo[2,2,1]heptan-2,3-dicarbonsäure (Endothall) | 0,2-2 |
| 1,1'-Dimethyl-4,4'-bipyridinium-Ion als Dichlorid- oder Dimethylsulfat-Salz (Paraquat) | 0,01-1 |
| N,N-Dimethylpiperidinium (Mepiquatchlorid) | 0,05-2 |
| 6,7-Dihydrodipyridol(1,2-α:2',1'-c)pyridilium dibromid (Diquat) | 0,1-1 |
| 2-sec-Butyl-4,6-dinitrophenol (Dinoseb) | 0,5-5 |

In den nachfolgenden Beispielen sind Herstellvorschriften für Verbindungen I und II, die in noch nicht offengelegten älteren Anmeldungen offenbart sind, beschrieben. Die in den Tabellen aufgeführten Wirkstoffe können durch entsprechende Abwandlung der Ausgangsstoffe erhalten werden.

### Herstellungsbeispiele:

A) Verbindungen mit A = I-4
   N-[4-Chlor-3-(4'-methyl-2-oxo-3'-oxa-cyclopentyliden-methyl]-phenyl-3,4,5,6-tetrahydrophthalimid 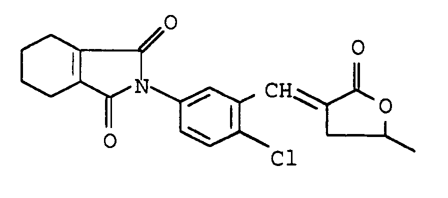
   a) Zu einer Mischung aus 7,4 g (0,054 mol) Kaliumcarbonat und 3,5 g (0,0135 mol) 18-Krone-6 in 10 ml abs. Tetrahydrofuran gibt man bei 0-5°C unter Rühren 7,1 g (0,03 mol) 4-Methyl-2-oxo-3-oxa-cyclopentyl-diethylphosphonat [Z. Naturforsch. B. 38B (4), 493 (1983)] in 8 ml abs. Tetrahydrofuran. Nach ca. 0,5 h versetzt man mit 5,0 g (0,027 mol) 2-Chlor-5-nitrobenzaldehyd in 6 ml abs. Tetrahydrofuran und läßt 15 h bei Raumtemperatur nachrühren. Zur Aufarbeitung gießt man auf 40 ml Eiswasser und extrahiert mehrfach mit Ether. Nach Waschen der org. Phase mit 10 %iger HCl und H₂O, Trocknen und Eindampfen des Lösungsmittels wird das Produkt an Kieselgel mit Toluol/Aceton (9:1) als Eluens getrennt. Man erhält so 1,7 g 2-(4'-Methyl-2'-oxo-3'-oxa-cyclopentyliden-methyl)-4-nitro-chlorbenzol (Fp.: 87-103°C, Isomerengemisch).
   b) Zu einer Mischung aus 3,0 g (0,054 mol) Eisenpulver, 7,5 ml Eisessig und 15 ml Methanol gibt man unter Rühren bei 60°C innerhalb 15 min 2,4 g (0,009 mol) 2-(4'-Methyl-2'-oxo-3'-oxa-cyclopentylidenmethyl)-4-nitro-chlorbenzol in 10 ml Eisessig und 10 ml Methanol. Nach beendeter Zugabe wird 30 min unter Rückfluß nachgerührt, auf RT abgekühlt, abfiltriert und das Lösungsmittel im Vakuum abgezogen. Der Rückstand wird mit Essigester aufgenommen, gewaschen, getrocknet und das Lösungsmittel entfernt. Man erhält so 2,3 g rohes 4-Amino-2-(4'-methyl-2'-oxo-3'-oxacyclopentylidenmethyl)-chlorbenzol, welches ohne weitere Aufarbeitung umgesetzt wird.
   c) 2,2 g (0,009 mol) 4-Amino-2-(4'-methyl-2'-oxo-3'-oxacyclopentylidenmethyl)-chlorbenzol, 1,4 g (0,009 mol) 3,4,5,6-Tetrahydrophthalsäureanhydrid und 25 ml Eisessig werden gemeinsam 2 h unter Rückfluß gerührt. Nach dem Abkühlen auf Raumtemperatur wird das Lösungsmittel abgezogen, der Rückstand in 100 ml Essigester aufgenommen, gewaschen, getrocknet und das Lösungsmittel entfernt. Man erhält so 3,5 g Rohprodukt, welches nach Chromatographie an Kieselgel mit Toluol/Aceton (98:2) 2,5 g N-[4-Chlor-3-(4'-methyl-2'-oxo-3'-oxa-cyclopentylidenmethyl)-phenyl]-3,4,5,6-tetrahydrophthalimid liefert. Fp.: 98-113°C.
   Weitere Beispiele für Wirkstoffe, die nach diesem Syntheseprinzip hergestellt worden sind, finden sich in Tabelle 9.
B) Verbindungen I mit A = I-5 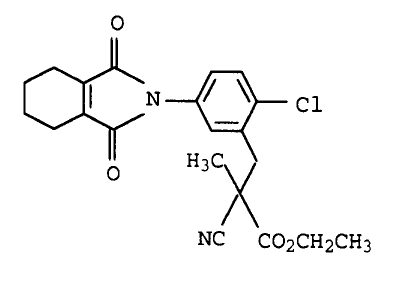
   a) 64,4 g 2-Chlorbenzylchlorid, 76,0 g 2-Cyanpropionsäure-ethylester, 58,0 g trockenes Kaliumcarbonat und 0,1 g 18-Krone-6 werden unter Ausschluß von Feuchtigkeit 3 Stunden am Rückfluß erhitzt. Nach Abfiltrieren und Destillation des Rückstands erhält man 69,5 g 2-Chlorbenzyl-methylcyanessigsäure-ethylester (Kp_{0,1}: 107-109°C).
   b) Zu 25,2 g des Esters gibt man 41,7 ml konz. Salpetersäure (d = 1,51) bei 0 bis 5°C in 30 Minuten und rührt weitere 30 Minuten. Man rührt in 400 ml Eiswasser ein und extrahiert zweimal mit je 70 ml Toluol. Nach Waschen mit 10 %iger Natriumbicarbonat-Lösung und Wasser, Verdampfen des Lösungsmittels und Umkristallisation aus 50 ml Diisopropylether gewinnt man 18,9 g (2-Chlor-5-nitro-benzyl)-methylcyanessigsäure-ethylester (Fp.: 72-74°C).
   c) Zu einer Mischung von 10,6 g Eisenpulver in 70 ml Methanol und 15 ml Eisessig tropft man bei Rückfluß in einer Stunde eine Lösung von 18,7 g der Nitroverbindung in 28 ml Methanol und 40 ml Eisessig. Nach weiteren 30 Minuten Sieden am Rückfluß wird abgesaugt und mit 50 ml Essigester nachgewaschen. Man rührt in 800 ml Wasser ein und extrahiert dreimal mit je 100 ml Essigester. Nach Trocknen und Verdampfen des Lösungsmittels im Vakuum isoliert man 15,5 g des oben bezeichneten Cyanessigsäure-ethylesters als Flüssigkeit.
   Nach diesem Syntheseprinzip können z.B. die in den Tabellen 10 und 11 aufgeführten Wirkstoffe hergestellt werden.
C) Verbindungen I mit A = I-6
   Variante 1 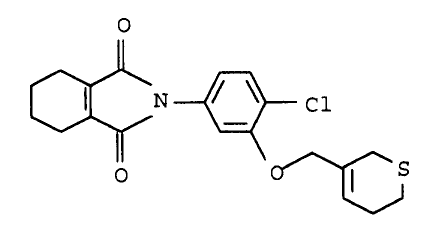 13,9 g N-(4-Chlor-3-hydroxyphenyl)-3,4,5,6-tetrahydrophthalimid, 8,2 g 3-Chlormethyl-5,6-dihydro-2H-thiopyran und 8,3 g Kaliumcarbonat wurden in 150 ml Acetonitril 5 Stunden unter Rückfluß erhitzt. Nach dem Abkühlen wurde abfiltriert, das Filtrat eingeengt und in 200 ml Methylenchlorid aufgenommen, 2mal mit 10 %iger Natronlauge, 3 mal mit Wasser gewaschen, getrocknet und eingeengt. Man erhielt 15,0 g N-[4-Chlor-3-(3-methoxy-5,6-dihydro-2H-thiopyranyl)-phenyl]-3,4,5,6-tetrahydrophthalimid (Fp.: 116-119°C).
   Variante 2 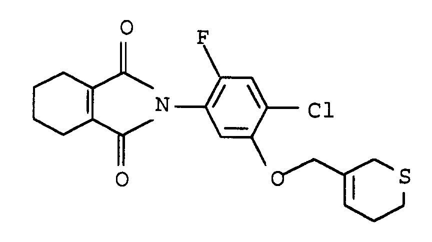
   a) 9,6 g 1-Chlor-4-fluor-5-nitrophenol, 8,2 g 4-Chlormethyl-5,6-dihydro-2H-thiopyran und 3,8 g Kaliumcarbonat wurden in 150 ml Acetonitril 5 Stunden unter Rückfluß erhitzt. Nach dem Abkühlen und Abfiltrieren wurde das Filtrat eingeengt und in 200 ml Methylenchlorid aufgenommen. Die organische Phase wurde dreimal mit je 50 ml Wasser gewaschen, getrocknet, eingeengt und mit Petrolether verrührt. Man erhielt 12,5 g 2-Chlor-4-fluor-5-nitro-(5,6-dihydro-2H-thiopyranyl-3-methyloxy)benzol (Fp.: 91-94°C).
   b) Zu einer Mischung von 6,7 g Eisenpulver in 50 ml Methanol und 7,5 ml Eisessig gab man bei Rückfluß portionsweise 12,2 g der obigen Nitroverbindung und erhitzte 2 Stunden am Rückfluß. Nach dem Abkühlen wurden 250 ml Wasser zugegeben und abgesaugt. Das Filtrat wurde 3 mal mit je 100 ml Essigester extrahiert, getrocknet und im Vakuum das Lösungsmittel verdampft. Nach Reinigung durch Chromatographie erhielt man 5,5 g 4-Chlor-2-fluor-5-(5,6-dihydro-2H-thiopyranyl-3-methyloxy)-anilin (Fp.: 73-74°C).
   c) 5,5 g des obigen Anilins und 3,0 g Cyclohexen-1,2-dicarbonsäureanhydrid wurden in 100 ml Eisessig 5 Stunden unter Rückfluß erhitzt. Nach dem Abkühlen gab man 50 ml Wasser zu und filtrierte ab. Der Niederschlag wurde mit Wasser gewaschen und getrocknet. Man erhielt 6,0 g N-[4-Chlor-2-fluor-5-(5.6-dihydro-2H-thiopyranol-3-methyloxy)-phenyl]-3,4,5,6-tetrahydrophthalimid (Fp.: 134-137°C).
   Weitere Beispiele für Wirkstoffe, die nach diesen Syntheseprinzipien hergestellt werden können, finden sich in Tabelle 12.
D) Verbindungen II 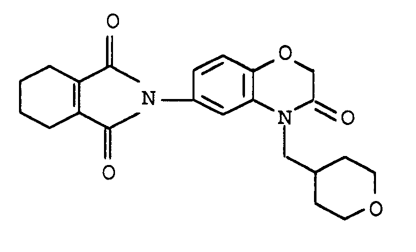
   a) 4,9 g (0,03 mol) 6-Amino-3,4-dihydro-2H-1,4-benzoxazin-3-on werden in 50 ml Dimethylformamid vorgelegt und bei 5°C 0,79 g (0,033 mol) Natriumhydrid zugegeben und 30 Minuten bei dieser Temperatur gerührt. Danach werden 5,9 g (0,033 mol) 3-Brommethyl-tetrahydropyran zugegeben, 3 Stunden bei 60°C gerührt, 200 ml Wasser zugegeben, zweimal mit 200 ml Methylenchlorid extrahiert, getrocknet und im Vakuum das Lösungsmittel verdampft. Man erhält 5 g (64 %) 4-(Tetrahydropyran-4-yl-methyl)-6-amino-3,4-dihydro-2H-1,4-benzoxazin-3-on (öl).
   b) 4,5 g (0,017 mol) des obigen Anilins und 2,7 g (0,018 mol) Cyclohexen-1,2-dicarbonsäureanhydrid werden in 70 ml Eisessig 3 Stunden bei 70°C gerührt. Nach dem Abkühlen gibt man 100 ml Wasser zu und filtriert ab. Der Niederschlag wird mit Wasser gewaschen und getrocknet. Man erhält 3,5 g (52 %) N-[4-(Tetrahydropyranyl-4-yl-methyl)-3,3-dihydro-2H-1,4-benzoxazin-3-on-6-yl]-3,4,5,6-tetrahydrophthalimid (Fp.: 187-189°C).
   Nach diesem Syntheseprinzip können die in den Tabellen 13 bis 15 dargestellten Wirkstoffe hergestellt werden.
   Beispiele für Wirkstoffe der Formeln I und II sind in den nachfolgenden Tabellen 4 bis 15 aufgeführt.
E) Verbindungen I mit A = I-1 und Q = O 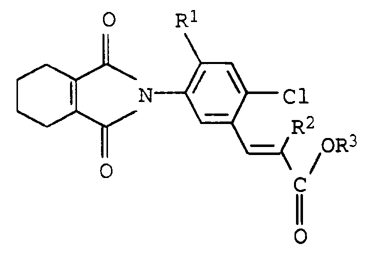 durch Wittig-Reaktion von 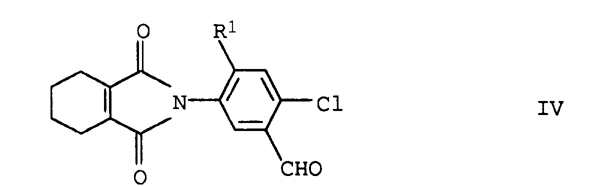 mit einem Phosphoran 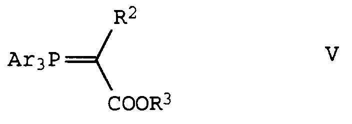 N-[5-(α-Bromacrylsäuremethylester)-4-chlor-2-fluorphenyl]-3,4,5,6-tetrahydrophthalimid
   4,60 g (15 mmol) N-(2-Fluor-4-chlor-5-formyl-phenyl)-3,4,5,6-tetrahydrophthalimid und 6,80 g (16,5 mmol) Carbomethoxybrommethylen-triphenylphosphoran wurden in 15 ml Methanol 15 min lang bei Raumtemperatur gerührt. Die Reaktionsmischung wurde auf 0°C gekühlt und das ausgefallene Produkt abfiltriert. Ausbeute: 70 %, Fp.: 155-157°C.
   N-[5-(α-Chloracrylsäureethylester)-4-chlor-2-fluor-phenyl]-3,4,5,6-tetrahydrophthalimid
   4,60 g (15 mmol) N-(2-Fluor-4-chlor-5-formyl-phenyl)-3,4,5,6-tetrahydrophthalimid und 6,30 g (16,5 mmol) Carboethoxychlormethylen-triphenyl-phosphoran wurden in 15 ml Ethanol 15 min lang bei Raumtemperatur gerührt. Anschließend wurde auf 0°C abgekühlt und das ausgefallene Produkt abfiltriert. Ausbeute: 68 %, Fp.: 104-107°C.
   N-[3-(α-Chloracrylsäureethylester)-4-chlor-phenyl]-3,4,5,6-tetrahydrophthalimid
   Zu 956 g (2,5 mol) Carboethoxychlormethylen-triphenylphosphoran in 1,3 l Ethanol wurden bei 30°C 651 g (2,25 mol) N-(4-Chlor-5-formyl-phenyl)-3,4,5,6-tetrahydrophthalimid portionsweise eingetragen. Nach beendeter Zugabe wurde noch 30 min gerührt. Anschließend wurde auf -10°C abgekühlt und das ausgefallene Produkt (3) abfiltriert. Ausbeute: 82 %, Fp.: 112-114°C.

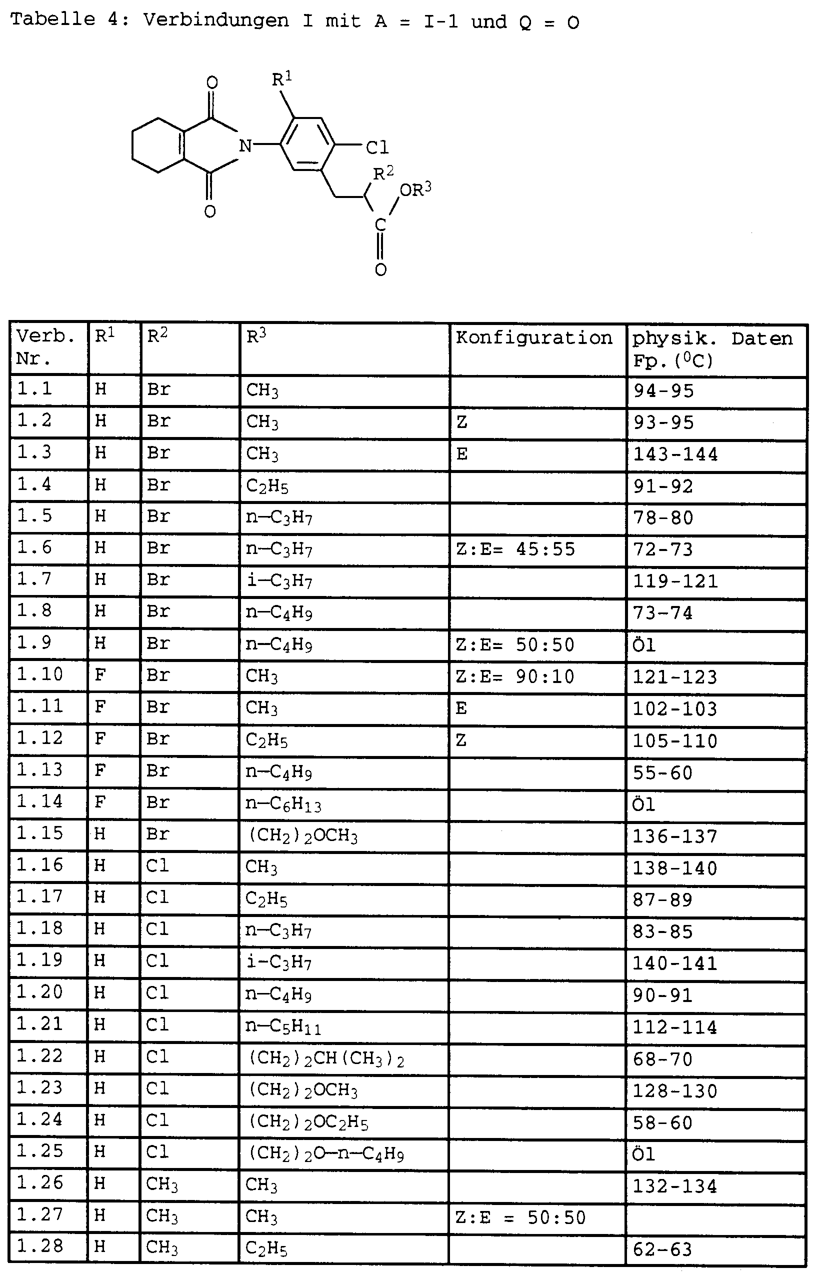

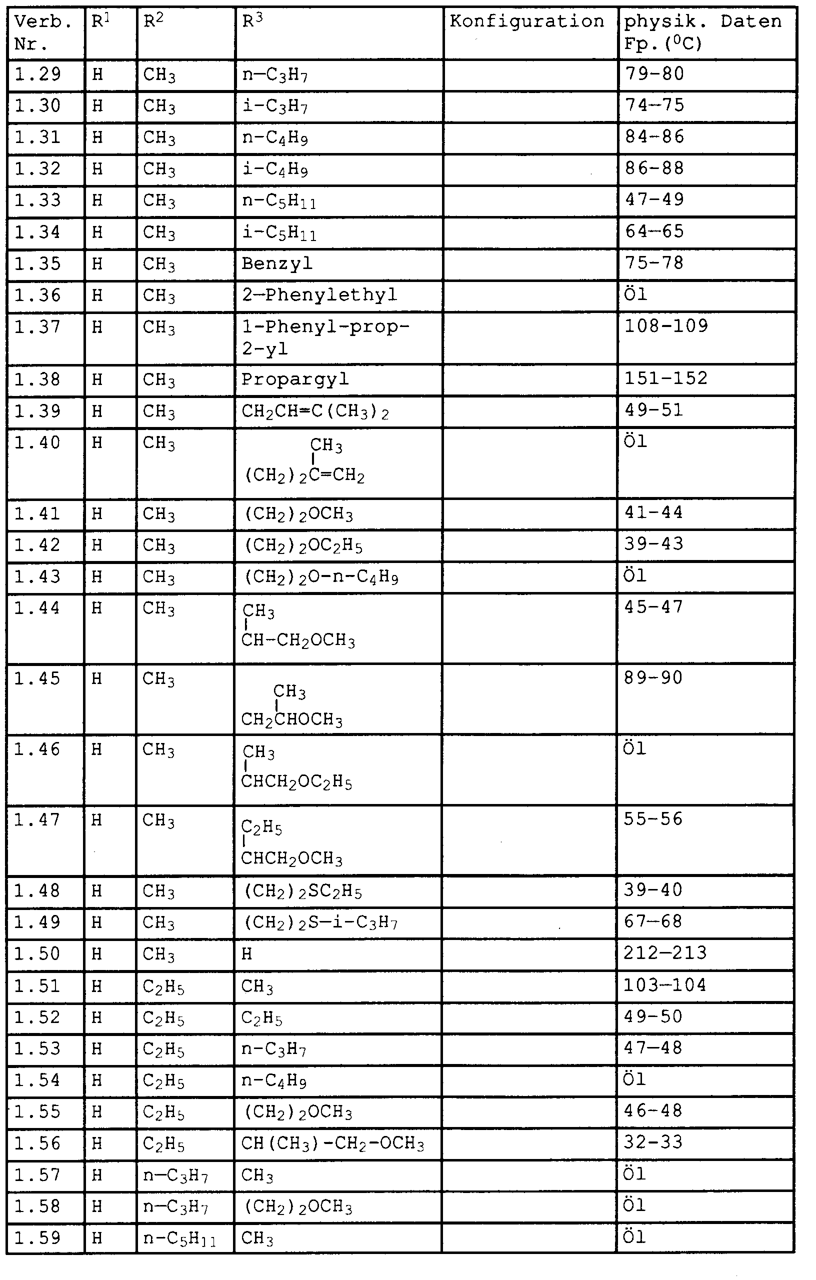

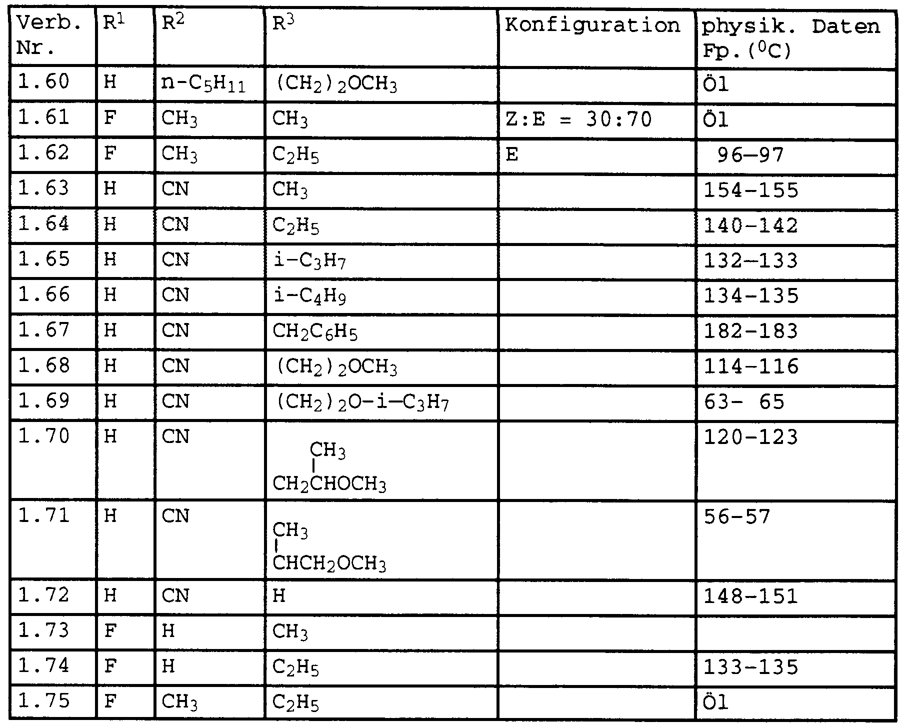

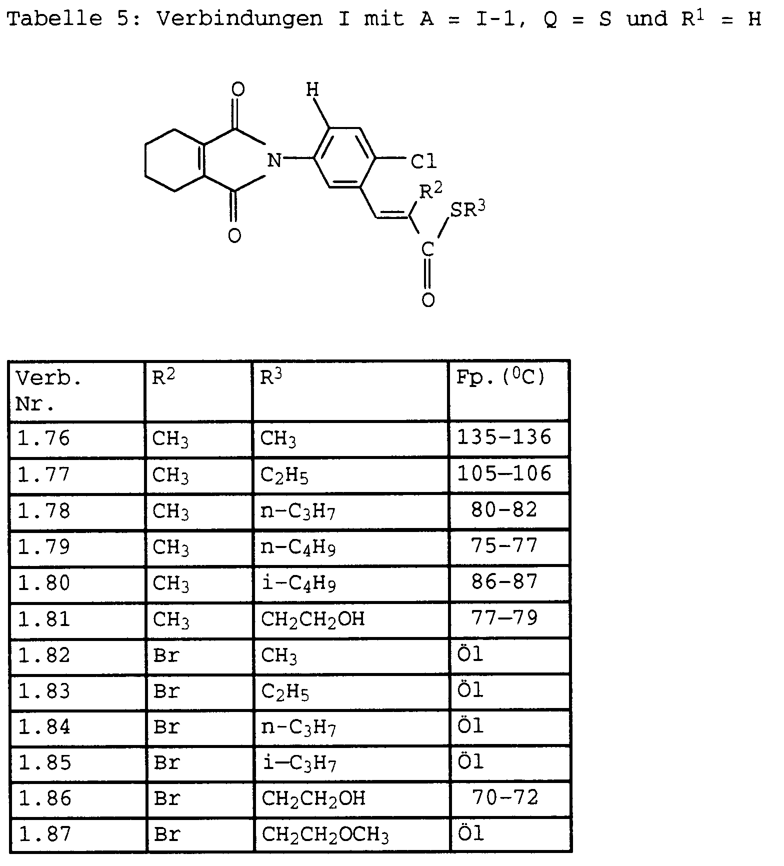

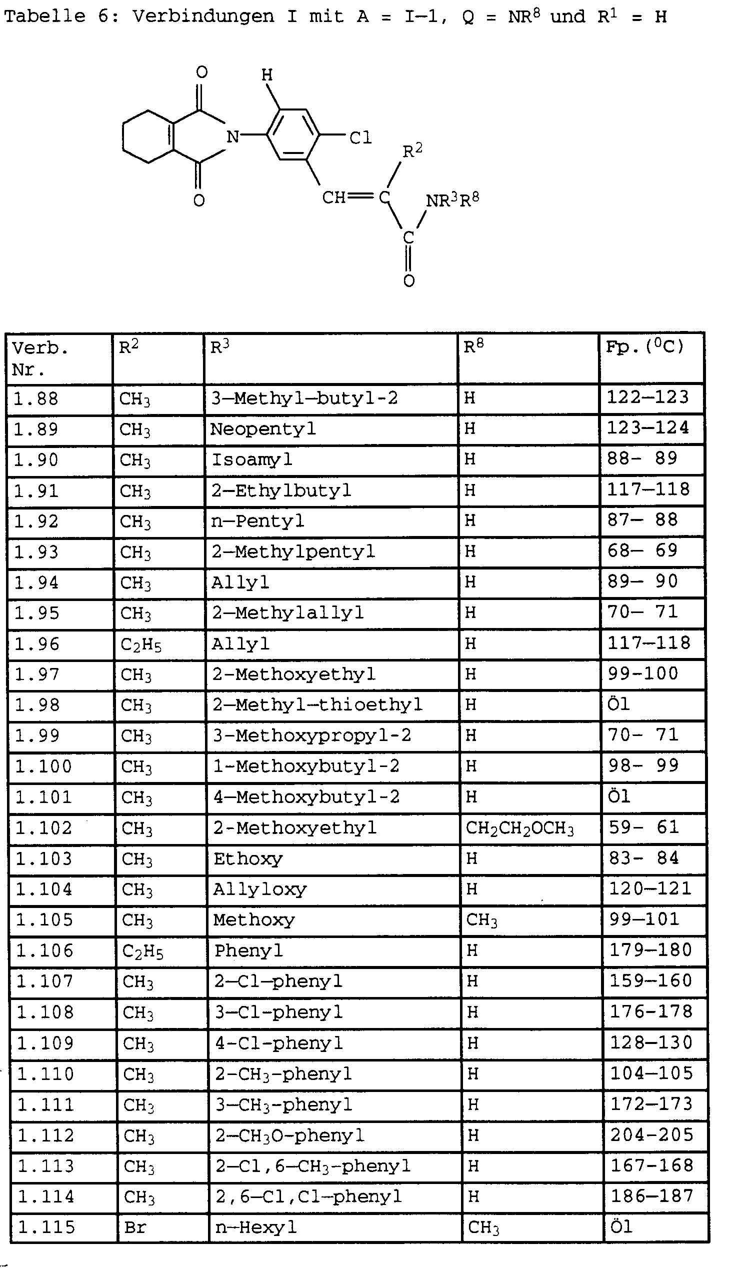

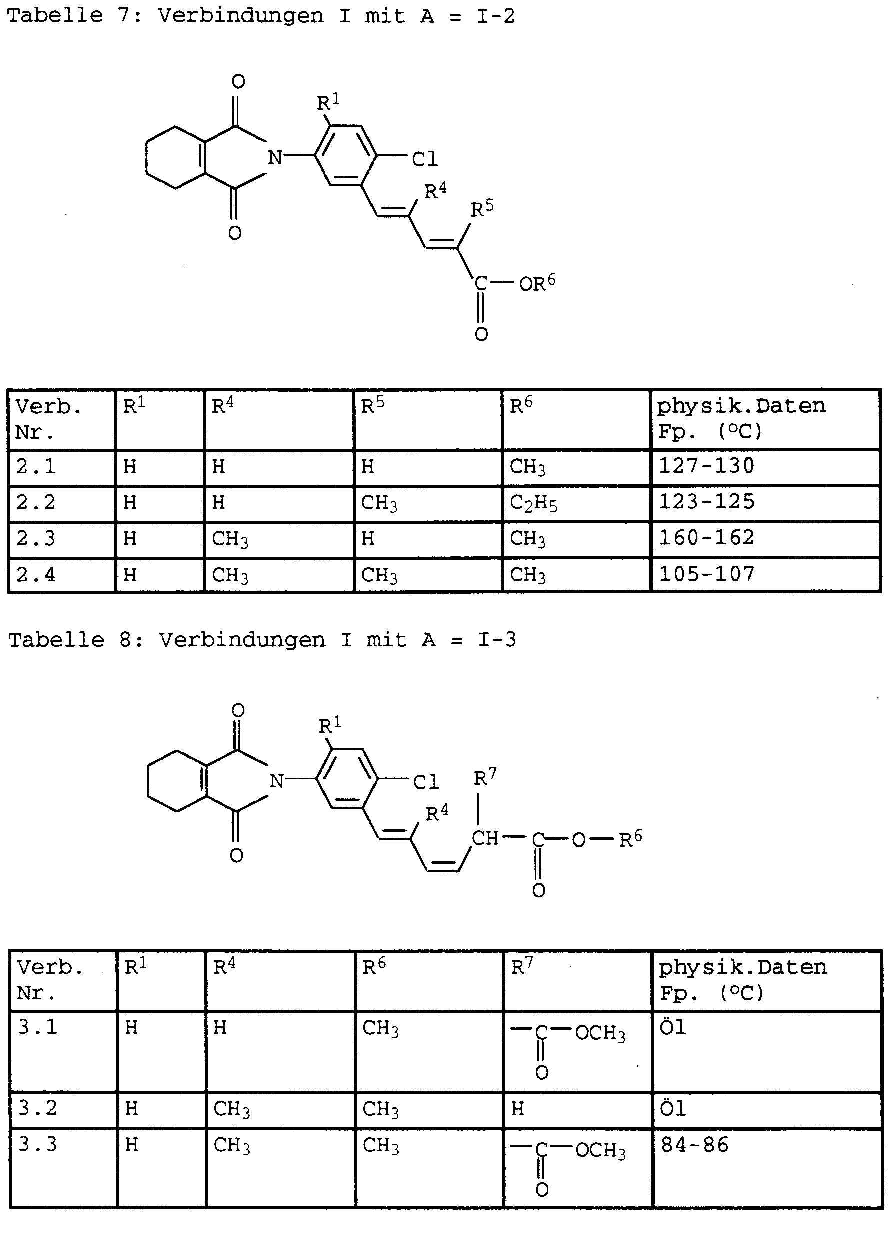

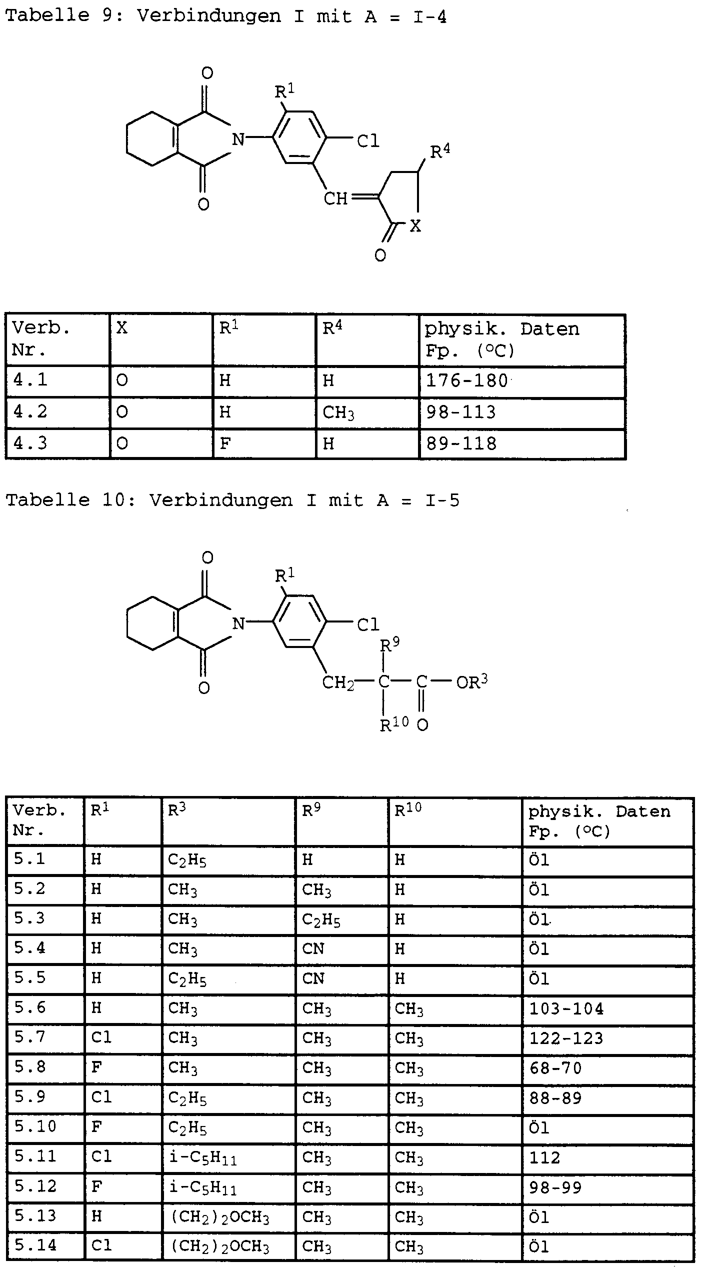

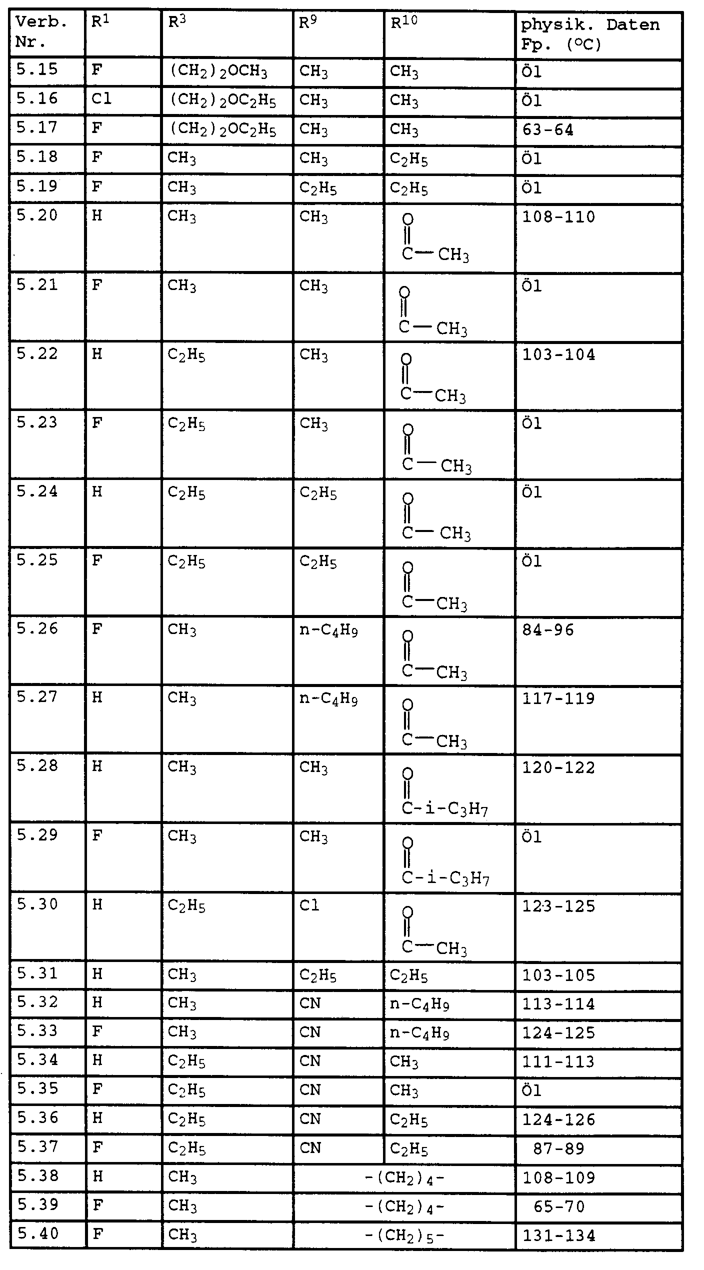

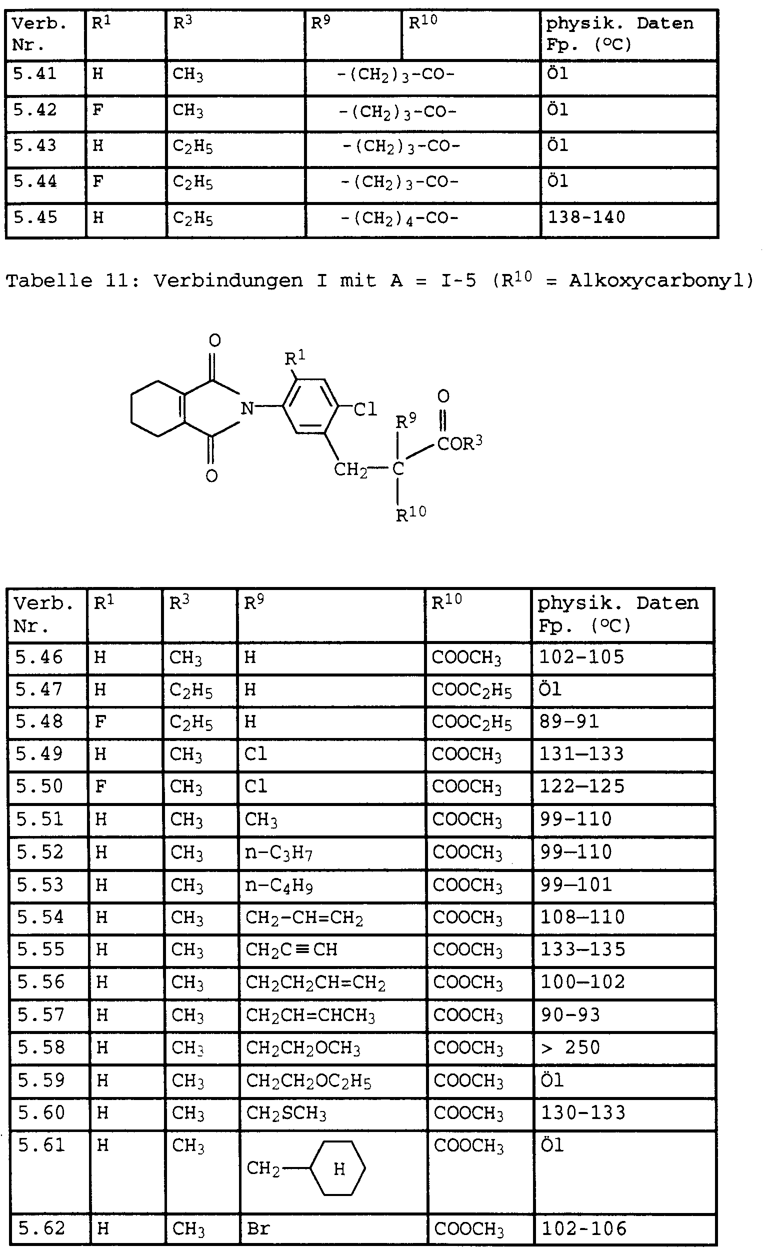

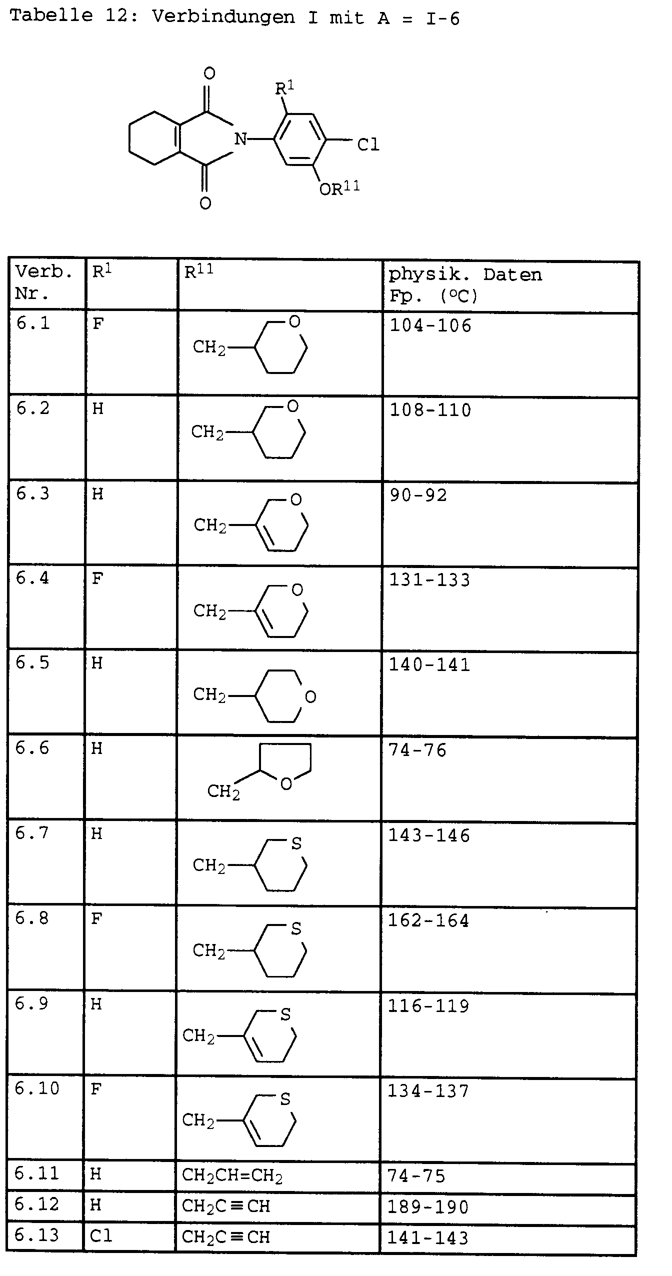

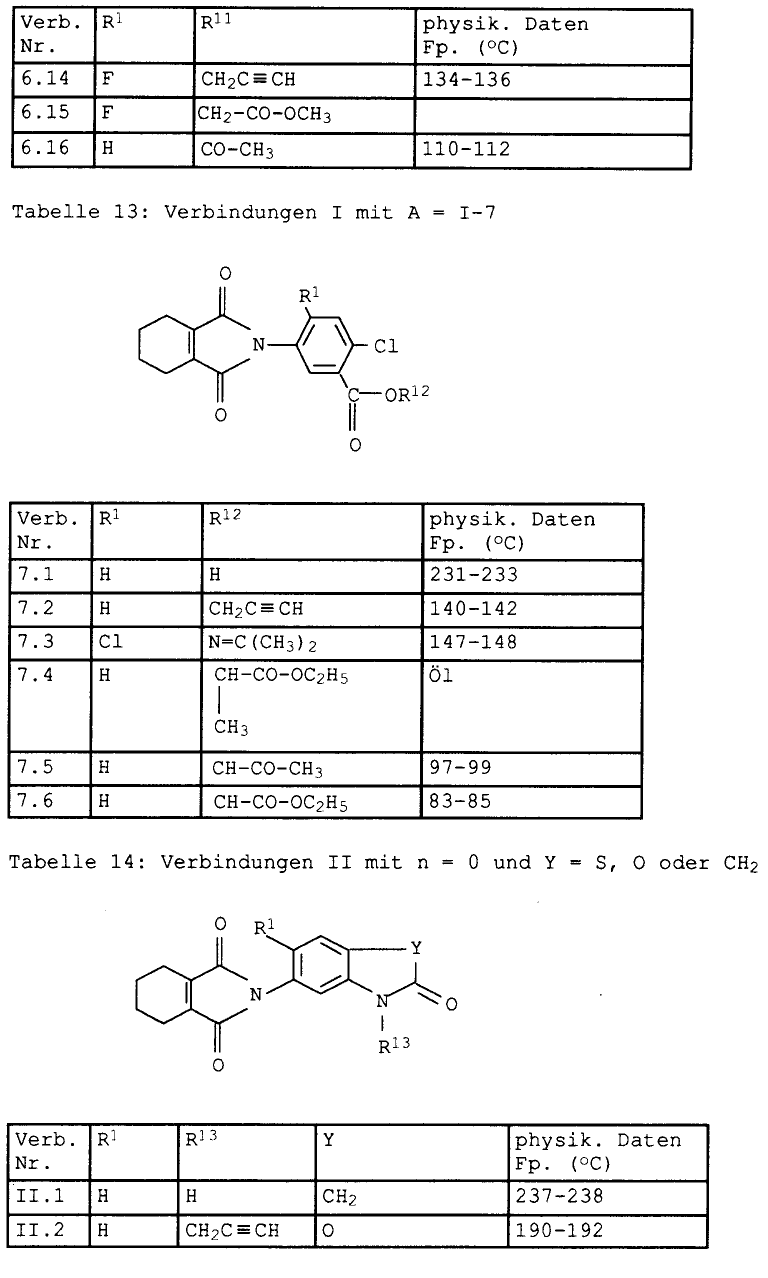

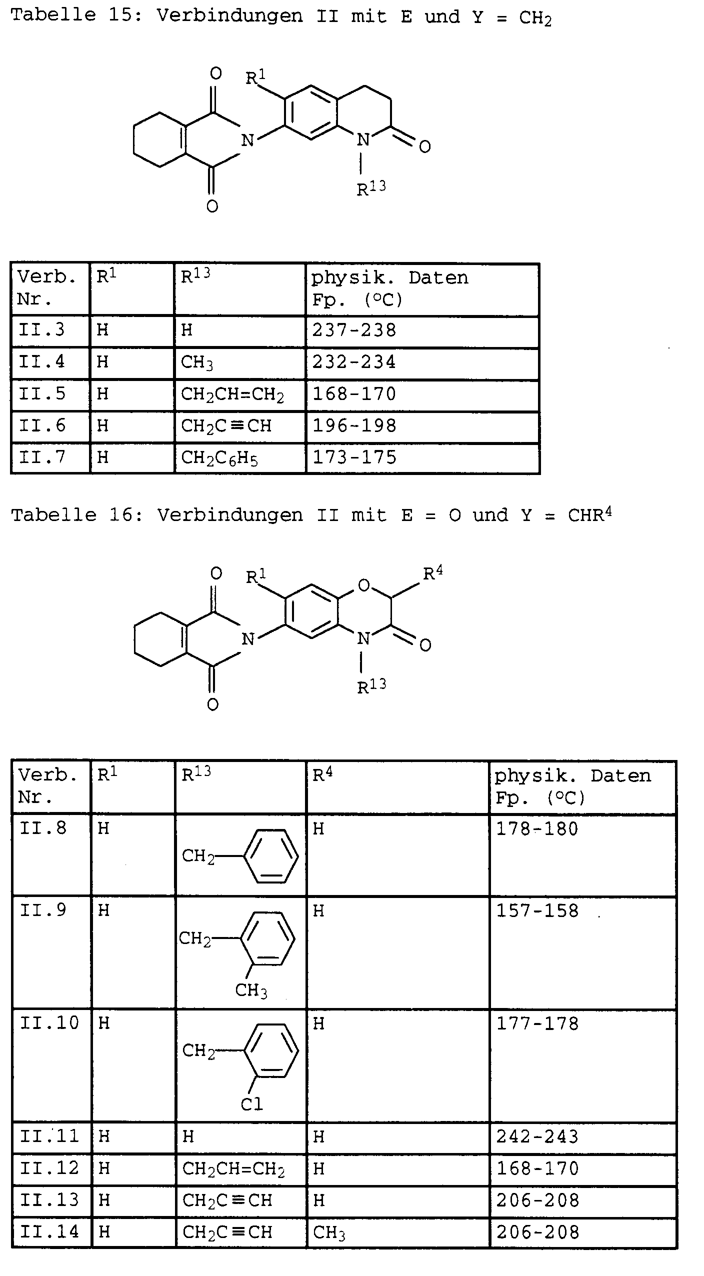

### Anwendungsbeispiele

Als Vergleichsmittel dienten
- VM1: N-Phenyl-N'-(1,2,3-thiadiazol-5-yl)-harnstoff und
- VM2: 6,7-Dihydrodipyridol(1,2-α:2',1'-c)pyridilium als Dibromid-Monohydrat-Salz
sowie die unter Beispiel G angegebenen Verbindungen VM3 - VM6 aus EP-A 207 894.

### Beispiel F

Junge Baumwollpflanzen (Sorte Delta Pine, Entwicklungsstadium: 5 - 6 entwickelte Laubblätter) wurden unter Gewächshausbedingungen angezogen (Tag/Nachttemperatur 23/16°C, relat. Luftfeuchte 50 - 70 %) und tropfnaß mit wäßrigen Aufbereitungen der angegebenen Wirkstoffe blattbehandelt. Die umgerechnete Wassermenge betrug 1000 l/ha. 12 Tage nach Wirkstoffapplikation wurde die Anzahl abgeworfener Blätter und der Grad der Entblätterung in % zur Kontrolle angegeben. Bei den unbehandelten Kontrollpflanzen trat kein Blattfall auf.

**Tabelle 17**

| Mittel, enthaltend Wirkstoff Nr. | Umgerechnete Aufwandmenge [kg/ha] | % Entblätterung |
|---|---|---|
| 1.1 | 0,062 | 26 |
| " | 0,125 | 61 |
| " | 0,250 | 80 |
| 1.26 | 0,062 | 67 |
| " | 0,125 | 79 |
| " | 0,250 | 89 |
| 1.17 | 0,062 | 55 |
| " | 0,125 | 84 |
| " | 0,250 | 89 |
| 6.1 | 0,062 | 83 |
| Vergl.mittel VM1 | 0,062 | 13 |
| " | 0,125 | 31 |
| " | 0,250 | 67 |
| Vergl.mittel VM2 | 0,250 | 11 |
| " | 0,500 | 27 |

Die Ergebnisse aus Beispiel F zeigen, daß die erfindungsgemäßen Mittel den handelsüblichen Wirkstoffen VM1 und VM2 deutlich überlegen sind und ihre gute Wirkung als Entblätterungsmittel auch bei geringeren Temperaturen entfalten.

### Beispiel G

Junge Baumwollpflanzen (Sorte Delta Pine, Entwicklungsstadium: 5 - 6 entwickelte Laubblätter) wurden unter Gewächshausbedingungen angezogen (Tag/Nachttemperatur 28/20°C, relat. Luftfeuchte 50 -70 %) und tropfnaß mit wäßrigen Aufbereitungen der angegebenen Wirkstoffe blattbehandelt. Die umgerechnete Wassermenge betrug 1000 l/ha. 3 Tage nach Wirkstoffapplikation wurde die Anzahl abgeworfener Blätter und der Grad der Entblätterung in % zur Kontrolle angegeben. Bei den unbehandelten Kontrollpflanzen trat kein Blattfall auf.

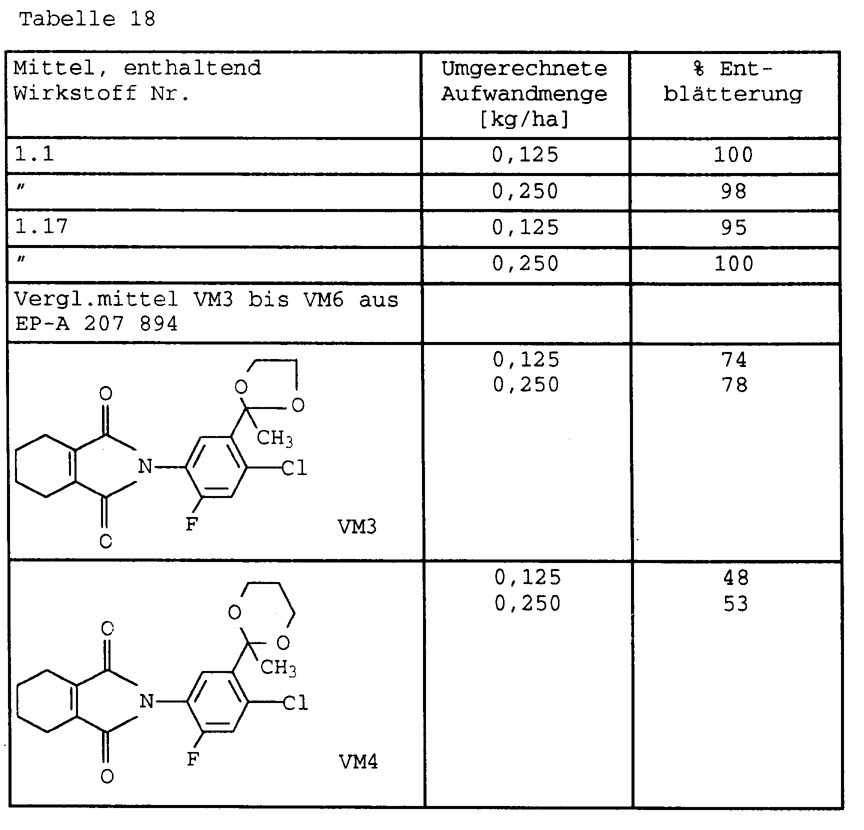

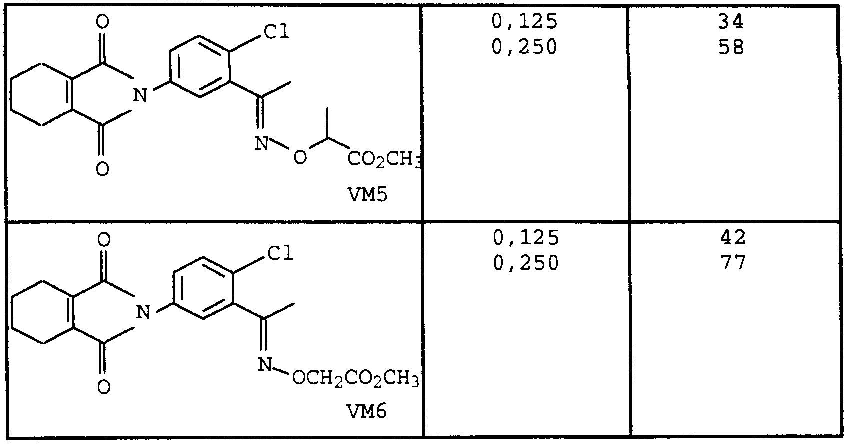

Die Ergebnisse zeigen, daß die erfindungsgemäßen Mittel den angegebenen Vergleichsmitteln aus EP-A 207 894 deutlich überlegen sind und ihre gute Wirkung als Entblätterungsmittel bei weit geringeren Aufwandmengen entfalten.

## Patentansprüche

1. Verwendung von Derivaten des N-Phenyl-3,4,5,6-tetrahydrophthalimids der allgemeinen Formeln I und/oder II 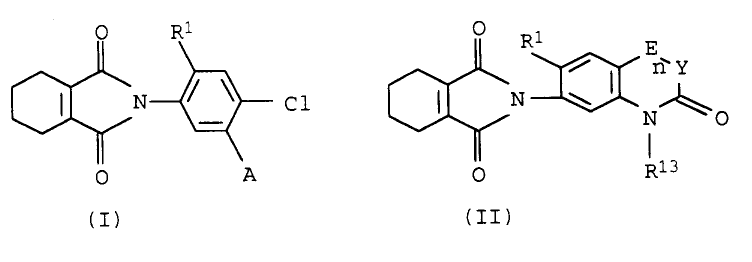 in denen die Substituenten folgende Bedeutung haben:
R¹ Wasserstoff, Fluor oder Chlor;
A eine Gruppe I-1 bis I-7 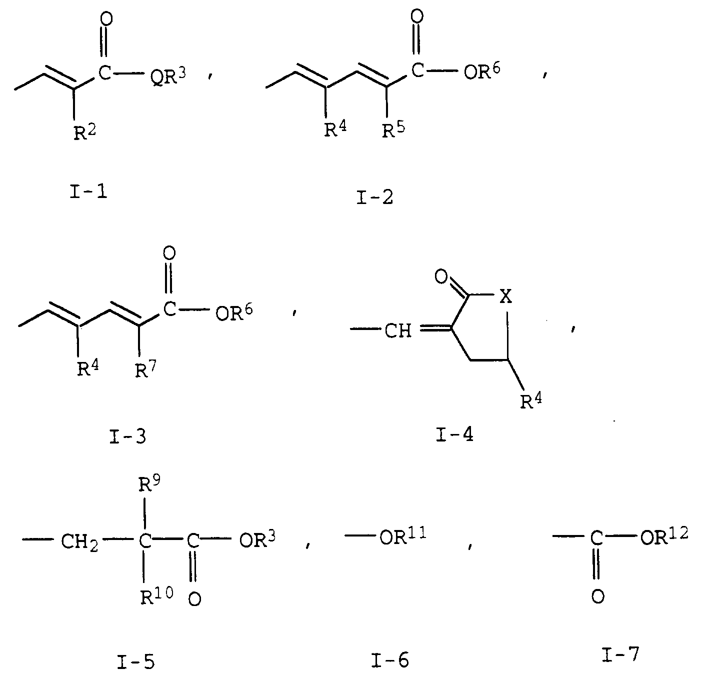
R² Wasserstoff, Chlor, Brom, Cyano oder C₁-C₆-Alkyl;
R³ Wasserstoff, C₁-C₈-Alkyl, C₂-C₈-Alkenyl, C₂-C₄-Alkinyl, C₁-C₄-Alkoxy-C₁-C₄-alkyl, C₁-C₄-Alkylthio-C₁-C₄-alkyl, Phenyl-C₁-C₃-alkyl und im Fall von Q = NR⁸ darüber hinaus C₁-C₄-Hydroxyalkyl, C₁-C₄-Alkoxy, C₂-C₄-Alkenyloxy, Phenyl, Phenyl substituiert durch Halogen, C₁-C₄-Alkyl oder -Alkoxy, C₁-C₄-Halogenalkyl oder -Halogenalkoxy;
R⁴, R⁵ Wasserstoff oder C₁-C₃-Alkyl;
E Sauerstoff oder Methylen;
X Sauerstoff oder Schwefel;
Q Sauerstoff, Schwefel oder NR⁸;
Y Sauerstoff, Schwefel, CHR⁴;
n die Zahl 0 oder 1;
R⁶ Wasserstoff, C₁-C₆-Alkyl, C₁-C₆-Alkoxyalkyl, C₁-C₆-Alkylthioalkyl oder C₅-C₆-Cycloalkyl;
R⁷ Wasserstoff oder C₁-C₄-Alkoxycarbonyl;
R⁸ C₁-C₈-Alkyl, C₃-C₄-Alkenyl, C₃-C₄-Alkinyl oder C₁-C₆-Alkoxyalkyl;
R⁹ Wasserstoff, Chlor, Brom, Cyano, C₁-C₄-Alkyl, C₃-C₄-Alkenyl, C₃-C₄-Alkinyl, C₁-C₆-Alkoxyalkyl, C₁-C₆-Alkylthioalkyl oder Cyclohexylmethyl;
R¹⁰ Wasserstoff, Halogen, Cyano, C₁-C₄-Alkyl, C₁-C₃-Alkylcarbonyl, C₁-C₄-Alkoxycarbonyl oder R⁹ und R¹⁰ zusammen eine C₄-C₅-Alkylen- oder C₄-C₅-Oxoalkylengruppe;
R¹¹ C₁-C₄-Alkyl, C₃-C₄-Alkenyl, C₃-C₄-Alkinyl, C₁-C₄-Alkylcarbonyl, C₁-C₄-Alkoxycarbonyl-C₁-C₄-alkyl, Tetrahydrofurfuryl, Dihydropyranylmethyl, Dihydrothiopyranylmethyl, Tetrahydropyranylmethyl oder Tetrahydrothiopyranylmethyl;
R¹² Wasserstoff, C₁-C₄-Alkyl, C₃-C₄-Alkinyl, C₁-C₄-Alkoxycarbonyl-C₁-C₄-alkyl oder -N=C(CH₃)₂;
R¹³ Wasserstoff, C₁-C₄-Alkyl, C₃-C₄-Alkenyl, C₃-C₄-Alkinyl, Benzyl, mit Halogen oder C₁-C₄-Alkyl ein- bis dreifach substituiertes
zur Desikkation und Abszission von Pflanzenorganen von Kulturpflanzen.

2. Verwendung von Derivaten des N-Phenyl-3,4,5,6-tetrahydrophthalimids der allgemeinen Formel I nach Anspruch 1, in der R¹ für Wasserstoff und A für die Gruppe I-1 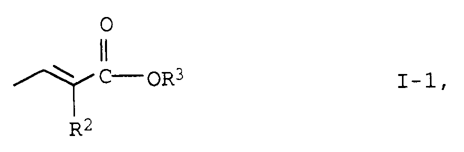 worin R¹ und R² die in Anspruch 1 genannte Bedeutung haben, stehen, zur Desikkation und Abszission von Pflanzenorganen.

3. Verwendung von Derivaten des N-Phenyl-3,4,5,6-tetrahydrophthalimids nach Anspruch 2, wobei R¹ für Chlor oder Brom und R² für Methyl oder Ethyl stehen.

4. Verwendung von Derivaten des N-Phenyl-3,4,5,6-tetrahydrophthalimids der allgemeinen Formel I nach Anspruch 1, in der R¹ für Wasserstoff und A für die Gruppe I-6
-OR¹¹ I-6
worin R¹¹ Tetrahydrofurfuryl, Dihydropyranylmethyl, Dihydrothiopyranylmethyl, Tetrahydropyranylmethyl oder Tetrahydrothiopyranylmethyl bedeutet, stehen, zur Desikkation und Abszission von Pflanzenorganen.

5. Verfahren zur Desikkation und Abszission von Pflanzenorganen, dadurch gekennzeichnet, daß man ein Derivat des N-Phenyl-3,4,5,6-tetrahydrophthalimids der allgemeinen Formeln I und/oder II gemäß den Ansprüchen 1 bis 4 auf Pflanzen oder deren Lebensraum einwirken läßt.

6. Verfahren nach Anspruch 5, dadurch gekennzeichnet, daß man 0,001 bis 5 kg/ha der Verbindungen I und/oder II auf die Pflanzen oder deren Lebensraum einwirken läßt.

## Claims

1. Use of a derivative of N-phenyl-3,4,5,6-tetrahydrophthalimide of the general formulae I and/or II 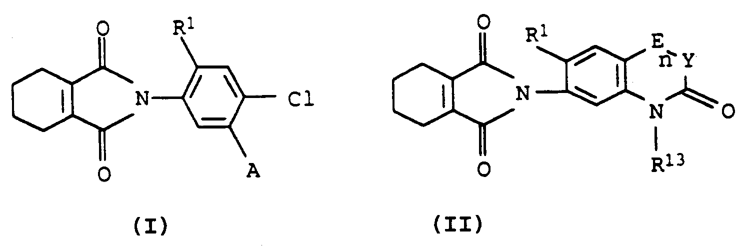 where
R¹ is hydrogen, fluorine or chlorine,
A is a group I-1 to I-7 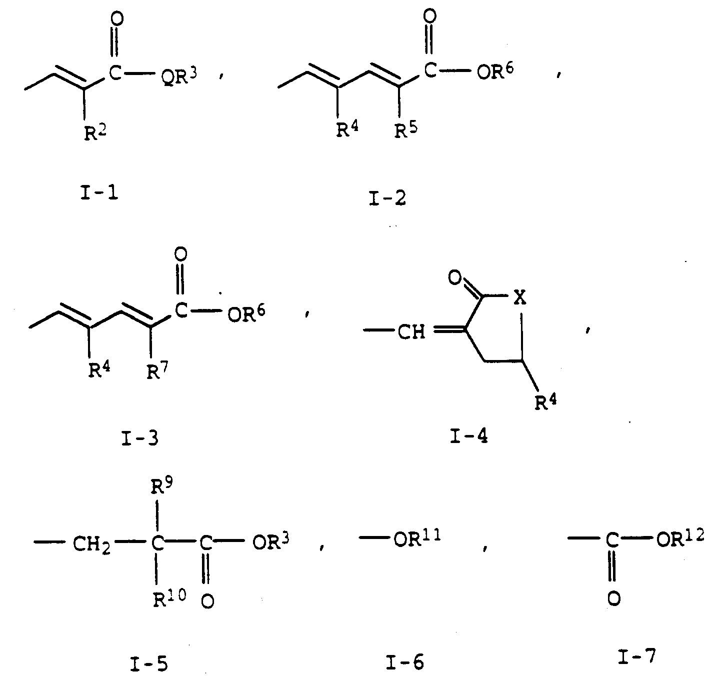 R² is hydrogen, chlorine, bromine, cyano or C₁-C₆-alkyl,
R³ is hydrogen, C₁-C₈-alkyl, C₂-C₈-alkenyl, C₂-C₄-alkynyl, C₁-C₄-alkoxy-C₁-C₄-alkyl, C₁-C₄-alkylthio-C₁-C₄-alkyl, phenyl-C₁-C₃-alkyl or, where Q is NR⁸, also C₁-C₄-hydroxyalkyl, C₁-C₄-alkoxy or C₂-C₄-alkenyloxy or phenyl which is unsubstituted or substituted by halogen, C₁-C₄-alkyl, C₁-C₄-alkoxy, C₁-C₄-haloalkyl or C₁-C₄-haloalkoxy,
R⁴ and R⁵ are each hydrogen or C₁-C₃-alkyl,
E is oxygen or methylene,
X is oxygen or sulfur,
Q is oxygen, sulfur or NR⁸,
Y is oxygen, sulfur or CHR⁴,
n is 0 or 1,
R⁶ is hydrogen, C₁-C₆-alkyl, C₁-C₆-alkoxyalkyl, C₁-C₆-alkylthioalkyl or C₅- or C₆-cycloalkyl,
R⁷ is hydrogen or C₁-C₄-alkoxycarbonyl,
R⁸ is C₁-C₈-alkyl, C₃- or C₄-alkenyl, C₃- or C₄-alkynyl or C₁-C₆-alkoxyalkyl,
R⁹ is hydrogen, chlorine, bromine, cyano, C₁-C₄-alkyl, C₃-or C₄-alkenyl, C₃- or C₄-alkynyl, C₁-C₆-alkoxyalkyl, C₁-C₆-alkylthioalkyl or cyclohexylmethyl,
R¹⁰ is hydrogen, halogen, cyano, C₁-C₄-alkyl, C₁-C₃-alkylcarbonyl or C₁-C₄-alkoxycarbonyl, or R⁹ and R¹⁰ together form a C₄- or C₅-alkylene or C₄- or C₅-oxoalkylene group,
R¹¹ is C₁-C₄-alkyl, C₃- or C₄-alkenyl, C₃- or C₄-alkynyl, C₁-C₄-alkylcarbonyl, C₁-C₄-alkoxycarbonyl-C₁-C₄-alkyl, tetrahydrofurfuryl, dihydropyranylmethyl, dihydrothiopyranylmethyl, tetrahydropyranylmethyl or tetrahydrothiopyranylmethyl,
R¹² is hydrogen, C₁-C₄-alkyl, C₃- or C₄-alkynyl, C₁-C₄-alkoxycarbonyl-C₁-C₄-alkyl or -N=C(CH₃)₂ and
R¹³ is hydrogen, C₁-C₄-alkyl, C₃- or C₄-alkenyl or C₃- or C₄-alkynyl or is benzyl which is unsubstituted or mono-substituted to trisubstituted by halogen or C₁-C₄-alkyl, for the desiccation and abscission of plant organs.

2. Use of a derivative of N-phenyl-3,4,5,6-tetrahydrophthalimide of the general formula I as claimed in claim 1, where R¹ is hydrogen and A is a group I-1 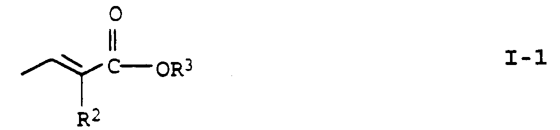 in which R¹ and R² have the meanings stated in claim 1, for the desiccation and abscission of plant organs.

3. Use of a derivative of N-phenyl-3,4,5,6-tetrahydrophthalimide as claimed in claim 2, wherein R¹ is chlorine or bromine and R² is methyl or ethyl.

4. Use of a derivative of N-phenyl-3,4,5,6-tetrahydrophthalimide of the general formula I as claimed in claim 1, where R¹ is hydrogen and A is a group I-6
-OR¹¹ I-6
in which R¹¹ is tetrahydrofurfuryl, dihydropyranylmethyl, dihydrothiopyranylmethyl, tetrahydropyranylmethyl or tetrahydrothiopyranylmethyl, for the desiccation and abscission of plant organs.

5. A method for the desiccation and abscission of plant organs, wherein a derivative of N-phenyl-3,4,5,6-tetrahydrophthalimide of the general formulae I and/or II as claimed in any of claims 1 to 4 is allowed to act on plants or their habitat.

6. A method as claimed in claim 5, wherein from 0.001 to 5 kg/ha of the compounds I and/or II are allowed to act on the plants or their habitat.

## Revendications

1. Utilisation de dérivés de N-phényl-3,4,5,6-tétrahydrophtalimide de formules générales I et/ou II 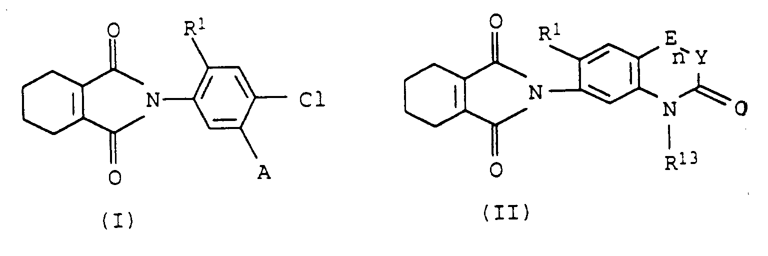 où les substituants ont la signification suivante:
R¹ hydrogène, fluor ou chlore;
A un groupe I-1 à I-7 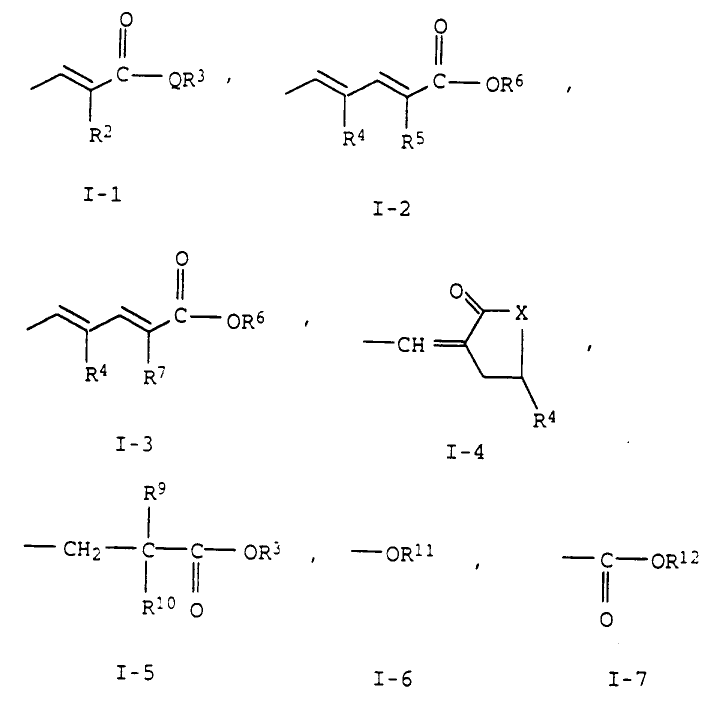
R² hydrogène, chlore, brome, cyano ou alkyle en C₁-C₆;
R³ hydrogène, alkyle en C₁-C₈, alcényle en C₂-C₈, alcynyle en C₂-C₄, alcoxy(C₁-C₄)-alkyle(C₁-C₄), alkylthio(C₁-C₄)alkyle(C₁-C₄), phényl-alkyle(C₁-C₃) et dans le cas où Q = NR⁸ en outre hydroxyalkyle en C₁-C₄, alcoxy en C₁-C₄, alcényloxy en C₂-C₄, phényle, phényle substitué par un halogène, alkyle en C₁-C₄ ou alcoxy en C₁-C₄, halogénoalkyle en C₁-C₄ ou halogénoalcoxy en C₁-C₄;
R⁴,R⁵ hydrogène ou alkyle en C₁-C₃;
E oxygène ou méthylène;
X oxygène ou soufre;
Q oxygène, soufre ou NR⁸;
Y oxygène, soufre, CHR⁴;
n le nombre 0 ou 1;
R⁶ hydrogène, alkyle en C₁-C₆, alcoxyalkyle en C₁-C₆, alkylthioalkyle en C₁-C₆ ou cycloalkyle en C₅-C₆;
R⁷ hydrogène ou alcoxycarbonyle en C₁-C₄;
R⁸ alkyle en C₁-C₈, alcényle en C₃-C₄, alcynyle en C₃-C₄ ou alcoxyalkyle en C₁-C₆;
R⁹ hydrogène, chlore, brome, cyano, alkyle en C₁-C₄, alcényle en C₃-C₄, alcynyle en C₃-C₄, alcoxyalkyle en C₁-C₆, alkylthioalkyle en C₁-C₆ ou cyclohexylméthyle;
R¹⁰ hydrogène, halogène, cyano, alkyle en C₁-C₄, alkylcarbonyle en C₁-C₃, alcoxycarbonyle en C₁-C₄ ou R⁹ et R¹⁰ représentent ensemble un groupe alkylène en C₄-C₅ ou oxoalkylène en C₄-C₅;
R¹¹ alkyle en C₁-C₄, alcényle en C₃-C₄, alcynyle en C₃-C₄, alkyl(C₁-C₄)carbonyle, alcoxy(C₁-C₄)carbonylalkyle(C₁-C₄), tétrahydrofurfuryle, dihydropyrannylméthyle, dihydrothiopyrannylméthyle, tétrahydropyrannylméthyle ou tétrahydrothiopyrannylméthyle;
R¹² hydrogène, alkyle en C₁-C₄, alcynyle en C₃-C₄, alcoxy(C₁-C₄)carbonylalkyle(C₁-C₄) ou -N=C(CH₃)₂;
R¹³ hydrogène, alkyle en C₁-C₄, alcényle en C₃-C₄, alcynyle en C₃-C₄, benzyle, benzyle substitué une à trois fois par un halogène ou un groupe alkyle en C₁-C₄,
pour la dessiccation et l'abscission d'organes végétaux des plantes cultivées.

2. Utilisation de dérivés de N-phényl-3,4,5,6-tétrahydrophtalimide de formule générale I selon la revendication 1, où R¹ désigne l'hydrogène et A représente le groupe I-1 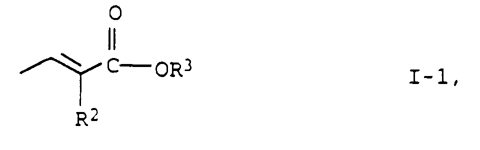 où R¹ et R² ont la signification indiquée dans la revendication 1, pour la dessiccation et l'abscission d'organes de plantes.

3. Utilisation de dérivés de N-phényl-3,4,5,6-tétrahydrophtalimide selon la revendication 2, où R¹ désigne le chlore ou le brome et R² représente un groupe méthyle ou éthyle.

4. Utilisation de dérivés de N-phényl-3,4,5,6-tétrahydrophtalimide de formule générale I selon la revendication 1, où R¹ désigne l'hydrogène et A représente le groupe I-6
-OR¹¹ I-6,
où R¹¹ représente un groupe tétrahydrofurfuryle, dihydropyrannylméthyle, dihydrothiopyrannylméthyle, tétrahydropyrannylméthyle ou tétrahydrothiopyrannylméthyle, pour la dessiccation et l'abscission d'organes de plantes.

5. Procédé pour la dessiccation et l'abscission d'organes végétaux, caractérisé par le fait qu'on fait agir un dérivé de N-phényl-3,4,5,6-tétrahydrophtalimide de formules générales I et/ou II selon les revendications 1 à 4 sur des plantes ou leur biotope.

6. Procédé selon la revendication 5, caractérisé par le fait qu'on fait agir 0,001 à 5 kg/ha des composés I et/ou II sur les plantes ou leur biotope.
